(19) Europäisches Patentamt
European Patent Office
Office européen des brevets

(11) Veröffentlichungsnummer: **0 088 345**
**B1**

(12) # EUROPÄISCHE PATENTSCHRIFT

(45) Veröffentlichungstag der Patentschrift: **17.09.86**

(51) Int. Cl.⁴: **C 07 C 143/78**, C 07 D 295/22, A 61 K 31/18, A 61 K 31/40

(21) Anmeldenummer: **83102004.5**

(22) Anmeldetag: **02.03.83**

(54) **2-Aminomethyl-6-sulfamoylphenolderivate, Verfahren zu ihrer Herstellung, ihre Verwendung sowie pharmazeutische Präparate auf Basis dieser Verbindungen.**

(30) Priorität: **06.03.82 DE 3208189**

(43) Veröffentlichungstag der Anmeldung: **14.09.83 Patentblatt 83/37**

(45) Bekanntmachung des Hinweises auf die Patenterteilung: **17.09.86 Patentblatt 86/38**

(84) Benannte Vertragsstaaten: **AT BE CH DE FR GB IT LI NL**

(56) Entgegenhaltungen:
**DE - A - 2 461 601**
**DE - A - 2 658 766**

**JOURNAL OF MEDICAL CHEMISTRY, Nr. 23, 1980, G.E. STOKKER et al., "2-(Aminomethyl)phenols, a new class of saluretic agents. 1. Effects of nuclear substitution", Seiten 1414-1427**
**Patent Abstracts of Japan, Band 3, Nr. 87, 25. Juli 1979, Seite 41C53**

(73) Patentinhaber: **HOECHST AKTIENGESELLSCHAFT, Postfach 80 03 20, D-6230 Frankfurt am Main 80 (DE)**

(72) Erfinder: **Englert, Heinrich Christian, Dr., Wielandstrasse 6, D-6238 Hofheim am Taunus (DE)**
Erfinder: **Mania, Dieter, Dr., Berliner Ring 5, D-6233 Kelkheim (Taunus) (DE)**
Erfinder: **Muschaweck, Roman, Dr., Heimchenweg 39, D-6230 Frankfurt am Main 80 (DE)**
Erfinder: **Hropot, Max, Dr., Friedrich-Stolz-Strasse 13, D-6093 Flörsheim am Main (DE)**

**EP 0 088 345 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 2-Aminomethyl-6-sulfamoylphenole der Formel I

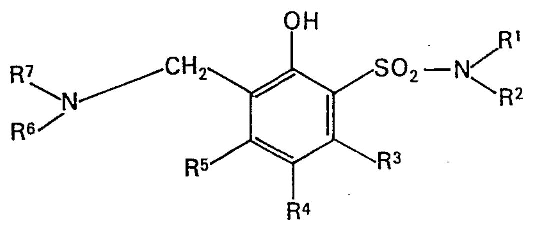

(I)

in welcher

$R^1$ und $R^2$ gleich oder verschieden sind und für Wasserstoff, Alkyl mit 1 bis 8 C-Atomen, Alkenyl mit 2 bis 8 C-Atomen, Alkinyl mit 2 bis 8 C-Atomen, Cycloakyl mit 3 bis 12 C-Atomen und bis zu 8 Ringgliedern oder Benzyl, das gegebenenfalls durch Alkyl mit 1 bis 4 C-Atomen Alkoxy mit 1 bis 4 C-Atomen oder Halogen substituiert ist, stehen,

$R^3$ und $R^5$ gleich oder verschieden sind und Wasserstoff, Halogen, Alkyl mit 1 oder 2 C-Atomen oder Alkoxy mit 1 oder 2 C-Atomen,

$R^4$ Halogen, Alkyl mit 1 bis 12 C-Atomen oder Cycloalkyl mit 3 bis 12 C-Atomen und bis zu 8 Ringgliedern bedeuten und

$R^6$ und $R^7$ gleich oder verschieden sind und für Wasserstoff oder Alkyl mit 1 bis 4 C-Atomen stehen,

wobei die Reste $R^1$ und $R^2$, $R^6$ und $R^7$ und/oder zwei der Reste $R^3$, $R^4$ und $R^5$ eine $-[CH_2]_m$-Kette mit $m = 3$ bis 6 bilden können, die gegebenenfalls durch 1 oder 2 Methylgruppen substituiert und die im Falle der Reste $R^1$, $R^2$, $R^6$ und $R^7$ auch durch 1 oder 2 Sauerstoffatome, Schwefelatome und/oder Iminogruppen unterbrochen sein kann, sowie deren physiologisch verträglichen Salze.

Hat eine der vorstehend definierten Cycloalkylgruppen eine C-Zahl, die über die Zahl der Ringglieder hinausgeht, so wird darunter eine Cycloalkylgruppe, die durch eine oder mehrere Alkylgruppen substituiert ist, oder eine ggf. alkylsubstituierte Cycloalkylalkylgruppe verstanden.

Aus J. med. Chem. 23 [1980] Seite 1414-1427 sind bereits in 6-Stellung substituierte 2-Aminomethylphenole bekannt. Die entsprechenden 4-Alkyl-6-Halogen-Derivate haben z.T. salidiuretische Eigenschaften von Art und Stärke, wie sie kurz und intensiv wirksame Diuretika, wie beispielsweise Furosemid, zeigen.

Der Ersatz der Halogenatome in 6-Position durch andere Substituenten bewirkt in den meisten Fällen eine Abschwächung oder gar den Verlust der salidiuretischen Wirksamkeit.

Es war daher äusserst überraschend, dass die erfindungsgemässen Verbindungen der Formel I an Hund oder Ratte eine salidiuretische Wirkung ausüben, die die Grössenordnung der Aktivität der oben beschriebenen Halogenderivate, insbesondere der Jodverbindung erreichen kann. Sie unterscheiden sich jedoch vorteilhaft dadurch, dass sie eine wesentlich geringere akute Toxizität aufweisen, wie dies an Ratten aufgezeigt werden kann.

Bevorzugt sind solche Verbindungen der Formel I, die dadurch gekennzeichnet sind, dass

$R^1$ und $R^2$ gleich oder verschieden sind und für Wasserstoff, Alkyl mit 1 bis 4 C-Atomen, Alkenyl oder Alkinyl mit 2 bis 4 C-Atomen und Benzyl, das gegebenenfalls durch Methyl, Methoxy oder Halogen substituiert ist, stehen,

$R^3$ und $R^5$ die oben genannte Bedeutung haben,

$R^4$ Halogen, Alkyl mit 1 bis 8 C-Atomen, Cycloalkyl mit 3 bis 10 C-Atomen und bis 7 Ringgliedern und

$R^6$ und $R^7$ gleich oder verschieden sind und Wasserstoff oder Alkyl mit 1 bis 2 C-Atomen bedeuten,

wobei die Reste $R^1$ und $R^2$, $R^6$ und $R^7$ und/oder zwei der Reste $R^3$, $R^4$ und $R^5$ eine oben definierte Polymethylenkette bilden können.

Insbesondere kommen von den erfindungsgemässen Verbindungen der allgemeinen Formel I diejenigen in Betracht, in denen

$R^1$ und $R^2$ gleiche oder verschiedene Reste Wasserstoff, Methyl, Ethyl oder beide zusammen $-[CH_2]_4-$ bedeuten,

$R^3$ und $R^5$ für Wasserstoff stehen

$R^4$ iso-Propyl, tert.-Butyl, n-Butyl, n-Propyl, sec.-Butyl, tert.-Amyl oder Cyclopentyl und

$R^6$ und $R^7$ Wasserstoff bedeuten,

wobei als besonders bevorzugte Verbindungen solche in Betracht kommen, in denen

$R^1$ und $R^2$ Wasserstoff oder zusammen $-[CH_2]_4-$,

$R^3$ und $R^5$ Wasserstoff,

$R^4$ iso-Propyl, tert.-Butyl, sec. Butyl und

$R^6$ und $R^7$ Wasserstoff bedeuten.

Die Erfindung betrifft weiterhin ein Verfahren zur Herstellung von Verbindungen der Formel I, das dadurch gekennzeichnet ist, dass

a) ein Phenol der Formel II

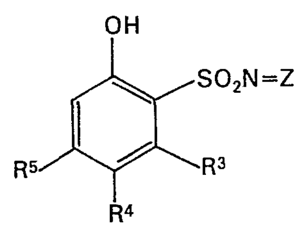

(II)

worin die Reste $R^3$ bis $R^5$ die oben genannten Bedeutungen haben und Z entweder für die beiden Reste $R^1$ und $R^2$, die die oben genannten Bedeutungen mit Ausnahme der von Wasserstoff haben oder für eine Schutzgruppe der Formel III steht,

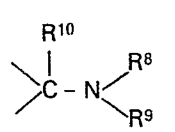

(III)

in der die Reste $R^{10}$ Wasserstoff oder Alkyl mit 1 bis 4 C-Atomen und $R^8$ und $R^9$ Alkyl mit 1 bis 4 C-Atomen bedeuten, mit einem N-Hydroxymethylcarboxamid der Formel IV,

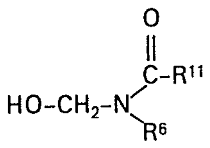 (IV)

in der $R^6$ die oben erwähnte Bedeutung hat und $R^{11}$ für Wasserstoff, Alkyl mit 1 bis 4 C-Atomen, Halogenalkyl mit 1-4 C-Atomen oder für Aryl mit 6 bis 10 C-Atomen steht oder in der $R_5$ oder der Rest $COR^{11}$ gemeinsam für den o-Phtaloylrest stehen zu Verbindungen der Formel V umsetzt

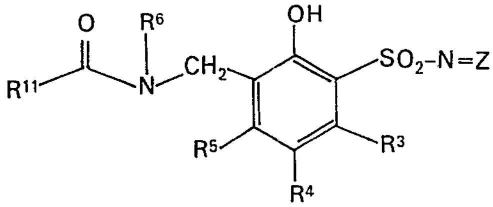 (V)

und den Rest $R^{11}$-C- sowie gegebenenfalls die Schutzgruppe Z hydrolytisch, abspaltet zu Verbindungen der allgemeinen Formel I,

b) ein Phenol der Formel II, in der die Reste $R^3$ bis $R^5$ sowie Z die oben genannten Bedeutungen haben, mit einem Amin der Formel VI

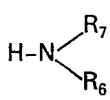 (VI)

in der $R^7$ und $R^6$ die oben genannte Bedeutung mit Ausnahme der von Wasserstoff besitzen, in Gegenwart von Formaldehyd umsetzt und gegebenenfalls die Sulfonamidschutzgruppe Z hydrolytisch abspaltet,

c) Phenole der Formel VII

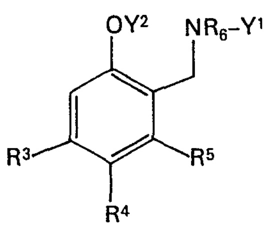 (VII)

in der $R^3$ bis $R^6$ die oben genannten Bedeutungen haben und $Y^1$ für den Rest $R^{11}$-CO- steht, wobei $R^{11}$ die oben genannte Bedeutung hat und $Y^2$ entweder Wasserstoff oder $Y^1$ bedeutet, chlorsulfoniert zu Verbindungen der Formel IX

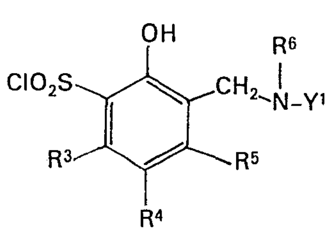 (IX)

und diese mit Aminen der Formel X

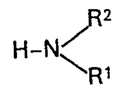 (X)

in der $R^1$ und $R^2$ die oben erwähnten Bedeutungen haben, zu Verbindungen der allgemeinen Formel XI umsetzt

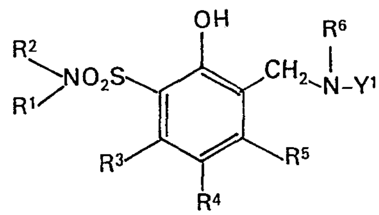 (XI)

und aus diesen den Rest $Y^1$ hydrolytisch abspaltet oder

d) Verbindungen der allgemeinen Formel XVIII

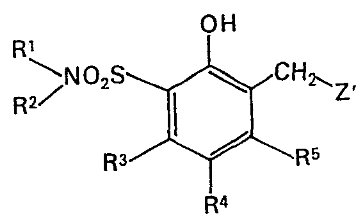 (XVIII)

in der $R^1$ bis $R^5$ die oben erwähnten Bedeutungen haben, wobei $R^1$ und $R^2$ aber auch gemeinsam für die bei Verfahrensvariante a erwähnte Sulfonamidschutzgruppe Z stehen können und Z′ für eine Fluchtgruppe wie beispielsweise Halogen, Tosylat, Dimethylamin oder Trimethylammonium steht, mit Aminen der allgemeinen Formel H-N=Y′, wobei Y′ entweder für die oben definierten Reste $R^6$ und $R^7$ steht oder für einen der Reste $R^6$ oder $R^7$ und eine Aminschutzgruppe wie etwa den Benzylrest oder den Acrylrest $CO-R^{11}$, wobei $R^{11}$ die unter Verfahrensvariante a aufgeführte Bedeutung hat, oder Y′ allein für eine Aminschutzgruppe wie beispielsweise den Diazorest oder den Phtaloylrest steht, zu den Verbindungen der allgemeinen Formel XIX umsetzt

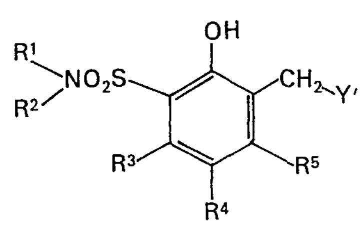 (XIX)

und aus diesen gegebenenfalls die Aminschutzgruppe und/oder die Sulfonamidschutzgruppe hydrolytisch oder hydrogenolytisch abspaltet.

Die Erfindung betrifft auch Verbindungen der allgemeinen Formel II

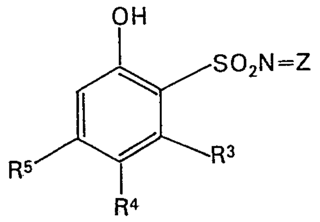

(II)

worin $R^3$ bis $R^5$ die oben angegebenen Bedeutungen haben und Z entweder für die oben definierten Reste $R^1$ und $R^2$ oder für die gemäss Verfahrensvariante a definierte Sulfonamidschutzgruppe steht.

Die Abspaltung des Restes

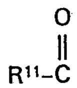

sowie gegebenenfalls der Schutzgruppe Z bei Verbindungen der allgemeinen Formel V gemäss Verfahrensvariante a wird im allgemeinen mit Hilfe einer Base oder einer Säure in Gegenwart von Wasser vorgenommen. Erfolgt die Abspaltung unter sauren Bedingungen, so findet vorzugsweise eine starke Mineralsäure wie Salzsäure, Brom- oder Jodwasserstoffsäure oder Schwefelsäure Verwendung. Wird dagegen alkalisch verseift, so werden vorteilhaft starke anorganische Basen wie Alkali oder Erdalkalibasen wie etwa Natriumhydroxid oder Bariumhydroxid verwendet, aber auch starke organische Basen wie quartäre Ammoniumhydroxide, z.B. Tetraethylammoniumhydroxid, können benützt werden. Als Lösemittel kann praktisch jedes, den Reaktionspartnern gegenüber inerte Lösungsmittel, wie z.B. Alkanole, vorzugsweise Ethanol oder, falls sauer hydrolysiert wird, Alkansäuren wie z.B. Essigsäure Verwendung finden. Pro verseifbare Gruppe muss wenigstens ein Äquivalent Wasser dem Reaktionsgemisch zugesetzt werden, meist wird jedoch ein grösserer Überschuss eingesetzt oder Wasser allein dient als Lösemittel, was insbesondere für alkalische Verseifungen vorteilhaft ist.

Die Reaktionstemperatur kann zwischen 20 und 150°C liegen, vorteilhaft wird bei Rückflusstemperatur des Lösemittels gearbeitet. Die Abspaltung der Sulfonamidschutzgruppe erfolgt dabei im allgemeinen wesentlich rascher als die Abspaltung des Restes

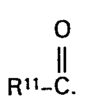

Nach saurer Verseifung fällt das Reaktionsprodukt meist sofort oder nach Abdampfen des Lösemittels in kristalliner Form als Säureadditionssalz an.

Bei alkalischer Verseifung der Verbindungen wird im allgemeinen nach Neutralisation überschüssiger Base sofort das freie Benzylamin I erzeugt.

Die Herstellung der Verbindungen der Formel V geschieht in an sich bekannter Weise dadurch, dass man die Phenole der allgemeinen Formel II nach Art der Tscherniac-Einhorn Reaktion mit N-Hydroxymethylcarboxamiden der allgemeinen Formel VI, vorzugsweise mit Z-Halogen-N-hydroxymethylacetamiden wie z.B. Z-Chlor-N-hydroxymethylacetamid unter Säurekatalyse umsetzt. Als Katalysatorsäuren eignen sich vor allem starke Mineralsäuren wie etwa Schwefelsäure oder Chlorwasserstoffsäure. Alle für Tscherniac-Einhorn Reaktionen gängigen Lösemittel können verwendet werden, besonders geeignet sind Alkansäuren, wie Essigsäure oder Propionsäure, aber auch überschüssige Mineralsäure als Lösemittel, wie z.B. reine konzentrierte Schwefelsäure, kann vorteilhaft sein. Die Umsetzungen werden zwischen 0° und 100°C vorgenommen, zur Vermeidung von Nebenprodukten vorteilhaft in einem Bereich von 0° bis 30°C.

Es zeigte sich insbesondere, dass derartige Amidoalkylierungen bei genau eingehaltener Reaktionszeit, die je nach Verbindung und Reaktionstemperatur zwischen 10 Minuten und einigen Stunden liegen kann, auch dann noch gut durchführbar sind, wenn verschärfte Reaktionsbedingungen (wie etwa konz. Schwefelsäure als Lösungsmittel), die sich unter Umständen als notwendig erweisen für einen raschen und vollständigen Umsatz, angewandt werden. Zu erwartende Nebenreaktionen wie etwa die Desalkylierung, insbesondere die Abspaltung der tert.-Butylgruppe (z.B. $R^4$ = tert.-Butyl in der allgemeinen Formel II oder V) können durch genau kontrollierte Reaktionsbedingungen auf ein Minimum reduziert werden.

Die Isolierung der Reaktionsprodukte geschieht am zweckmässigsten dadurch, dass man ein Nichtlösemittel, wie etwa Wasser, dem Reaktionsgemisch beifügt, das Produkt fällt dann in der Regel sofort kristallin an und kann nach Umkristallisation aus einem geeigneten Lösemittel oder in vielen Fällen ohne weitere Reinigung weiterverarbeitet werden.

Eine Methode zur Herstellung der als Ausgangsverbindungen für die Verfahrensvarianten a und b benützten Phenole der allgemeinen Formel II besteht z.B. in der Etherspaltung von Anisolen der allgemeinen Formel XII oder anderen Alkylphenolethern, die anstatt der $OCH_3$-Gruppe eine O-($C_2$-$C_6$)-Alkylgruppe tragen.

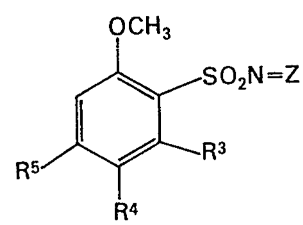

(XII)

Sie wird in an sich bekannter Weise durch Einwirken von Mineralsäuren wie Jodwasserstoffsäure oder von Lewissäuren wie Aluminiumchlorid oder Bortribromid in geeigneten Lösemitteln wie z.B. Methylenchlorid oder Chloroform vorgenommen.

Für den Fall, dass Z die Bedeutung einer Schutzgruppe der allgemeinen Formel III hat, können die Anisole XII wie folgt nach einer Sequenz von Standardmethoden aus Anisolen XIII hergestellt werden:

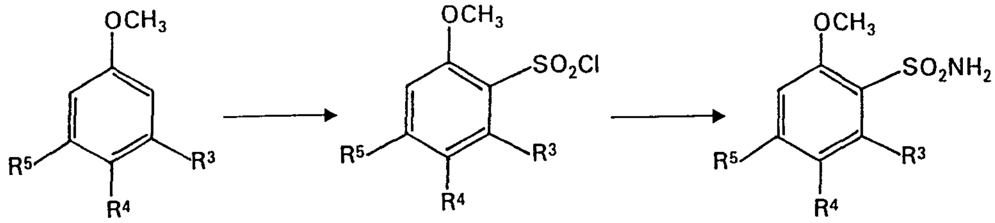

(XIII) (XIV) (XV)

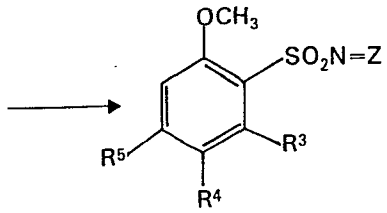

(XII)

Durch Einwirkung von Chlorsulfonsäure auf die Anisole XIII werden in an sich bekannter Weise (Houben-Weyl, Methoden der organischen Chemie, Band X, S. 563 ff, G. Thieme Verlag Stuttgart 1955) die Sulfochloride XIV erhalten und aus diesen durch Einwirkung von Ammoniak die Sulfonamide XV. Analog wie in der DE-OS 2 658 766 oder in DE-OS 24 61 601 beschrieben, werden diese durch Einwirkung von substituierten Formamiden, z.B. von Dimethylformamid in Gegenwart eines die Wasserabspaltung begünstigenden Reagens wie Thionylchlorid oder Phosphoroxychlorid zu den geschützten Sulfonamiden XII umgesetzt. Anstelle der Formamide können auch deren Acetale eingesetzt werden wie z.B. Dimethylformamiddimethylacetal, in diesem Fall läuft die Reaktion dann meist ganz ohne Zusatz eines Kondensationsmittels ab.

Für den Fall, dass Z für die beiden Reste $R^1$ und $R^2$ steht, wobei sie die oben erwähnten Bedeutungen mit Ausnahme der von Wasserstoff haben, werden die Anisole XII aus den Sulfochloriden XIV und den Aminen der Formel X in bekannter Weise hergestellt.

Die Anisole XIII werden durch Standardmethoden (Organikum, VEB Deutscher Verlag der Wissenschaften; Berlin 1971, S. 222) aus Phenolen hergestellt.

Verbindungen der Formel I, in denen sowohl $R^7$ als auch $R^6$ Alkylreste sind oder cyclisch miteinander verbunden sind, wie eingangs definiert wurde, werden vorteilhaft nach Verfahrensvariante b hergestellt, indem man die Phenole II nach Art der Mannich-Reaktion mit Aminen der allgemeinen Formel VI umsetzt. Formaldehyd wird dabei bevorzugt in Form einer wässrigen Lösung eingesetzt, es können jedoch auch alle anderen gängigen Varianten der Mannich-Reaktion zur Anwendung kommen, wie z.B. der Einsatz von Paraformaldehyd. Auch das Lösungsmittel kann in weiten Grenzen variiert werden, besonders geeignete Lösemittel sind Alkanole, wie etwa Methanol oder Ethanol. Man arbeitet in einem Temperaturbereich von 40° bis 150°C, bevorzugt jedoch in einem Bereich von 60° bis 100°C. Die Reaktionszeit hängt entscheidend von der Temperatur ab, im allgemeinen sind die Umsetzungen nach wenigen Stunden beendet. Man isoliert die Produkte bevorzugt durch Abdampfen des Lösemittels und überschüssiger Reagentien. Wurden Phenole II mit Z in der Bedeutung der oben erwähnten Sulfonamidschutzgruppe eingesetzt, so schliesst sich eine Hydrolyse wie unter Verfahrensvariante a beschrieben an. Die Produkte fallen meist als hochviskose Öle an, die entweder durch Kristallisation aus einem geeigneten Lösemittel gereinigt werden oder in ein kristallines Säureadditionssalz überführt werden können.

Werden die Verbindungen der Formel I nach der Verfahrensvariante c hergestellt, so wird die Abspaltung der Schutzgruppe Y' aus den Verbindungen der Formel XI unter denselben Bedingungen durchgeführt, die auch für die Verseifung von Verbindungen der Formel V unter Verfahrensvariante a angegeben wurden.

Die Sulfonamide XI werden aus den Sulfochloriden IX und den Aminen X nach Standardmethoden hergestellt. Im allgemeinen empfiehlt sich der Zusatz eines Säurefängers wie z.B. schwache organische Base wie etwa Triethylamin oder Pyridin, vorteilhaft wird oft ein zweites Äquivalent des Amins X zugesetzt, um die bei dieser Reaktion freigesetzte Salzsäure abzufangen. Als Lösemittel dienen alle den Reaktionspartnern gegenüber inerten Lösemittel, wie z.B. Ketone, Ester oder Ether, als besonders vorteilhaft hat es sich erwiesen, zu dem in einem Keton, wie Aceton gelösten Amin X das Sulfochlorid IX entweder gelöst oder in fester Form langsam und portionsweise zuzugeben. Auf diese Weise lassen sich polymere Nebenprodukte, die aus dem Sulfochlorid IX unter Basenkatalyse leicht entstehen, vermeiden, da stets ein grosser Überschuss an Amin X vorliegt. Weniger vorteilhaft verläuft deshalb die ansonsten gebräuchlichere Methode, ein Sulfochlorid vorzulegen und das Amin zuzusetzen.

Die Reaktionstemperatur kann zwischen 0 und 150°C liegen, im allgemeinen empfiehlt es sich, einen Bereich von 20 bis 50°C einzuhalten. Da die Reaktion unter Umständen exotherm verläuft, ist ein Kühlen des Reaktionsgemisches eventuell von Vorteil.

Die Reaktionsprodukte werden im allgemeinen durch Abdampfen des Lösemittels oder durch Zusatz eines Nichtlösemittels wie etwa Wasser kristallin isoliert. Sie bedürfen in den meisten Fällen einer weiteren Reinigung, in vielen Fällen genügt eine Umkristallisation aus einem organischen Lösemittel, vorteilhaft sind Alkanole wie n-Butanol, Isopropanol oder Ethanol.

Die Sulfochloride IX werden aus den Phenolen VII in an sich bekannter Weise hergestellt. Dabei ist es nicht unbedingt erforderlich, dass auch die phenolische Gruppe geschützt vorliegt, auch die freien Phenole lassen sich vorteilhaft einsetzen ($Y^2$ = H in der Formel VII). Die Umsetzung wird durch Einwirkung eines Sulfonierungsmittels, wie Schwefelsäure oder Chlorsulfonsäure, mit nachfolgender Einwirkung eines Chlorierungsmittels, wie etwa Sulfurylchlorid, Thionylchlorid oder Chlorsulfonsäure, erzielt. Vorzugsweise findet ein Verfahren Anwendung, bei dem man 2 oder mehr Äquivalente Chlorsulfonsäure auf das Phenol VII einwirken lässt. Man verwendet die für Chlorsulfonierungen üblichen Lösemittel, wie etwa Chlorkohlenwasserstoffe (z.B. Chloroform oder Methylenchlorid) oder arbeitet, was von besonderem Vorteil ist, ganz ohne Lösemittel bzw. verwendet überschüssiges Chlorierungsmittel wie die Chlorsulfonsäure als Lösemittel. Die Reaktionstemperatur lässt sich in weiten Grenzen variieren, z.B. zwischen -20° und +50°C, bevorzugt wird ein Temperaturbereich von 0° bis +20°C.

Das Reaktionsprodukt wird meist durch Zugabe eines Nichtlösemittels wie Eiswasser, das gleichzeitig die Zerstörung überschüssigen Chlorierungs- bzw. Chlorsulfonierungsmittels bewirkt, isoliert. Eine weitere Reinigung ist meist nicht nötig bzw. lässt sich, falls erforderlich, durch Umkristallisation, etwa aus unpolaren organischen Lösemitteln, wie Toluol, leicht bewerkstelligen.

Die Herstellung der Phenole VII erfolgt durch Acylierung von Salicylaminen XVI mit geeigneten Acylierungsmitteln

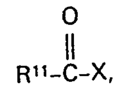

wobei $R^{11}$ die oben erwähnte Bedeutung hat und X für eine bei derartigen Reaktionen übliche Fluchtgruppe, wie etwa Halogen, steht.

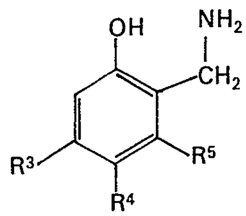

(XVI)

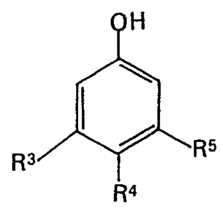

(XVII)

Die Salicylamine XVI lassen sich nach Standardmethoden herstellen.

Die Phenole VIII (mit $Y^2$ = H) sind auch direkt aus Phenolen XVII durch die Tscherniac-Einhorn-Reaktion unter Verwendung der Hydroxymethylcarboxamide der Formel IV herstellbar.

Die nach den verschiedenen Verfahrensvarianten hergestellten Verbindungen der Formel I fallen entweder als freie Basen oder als Säureadditionssalze an. Um aus einem Säureadditionssalz die freie Base zu erzeugen, ist es notwendig, das Salz mit wenigstens einem Äquivalent einer Base zu behandeln. Es kommen dabei sowohl organische als auch anorganische Basen in Frage, z.B. Triethylamin, Tetraethylammoniumhydroxid oder Piperidin bzw. Lithium-, Natrium-, Kaliumhydroxid, Natriumhydrogencarbonat, Natriumcarbonat und ähnliche. Vorteilhaft wird bei dieser Reaktion das Säureadditionssalz in gelöster Form eingesetzt, z.B. in Alkanolen wie Methanol oder Ethanol oder, was sich als besonders günstig erwies, in Form von wässrigen Lösungen, bei denen der Zusatz von anorganischer Base wie etwa Natriumhydroxid ein Ausfällen von kristalliner freier Base I bewirkt.

Umgekehrt werden Säureadditionsprodukte mit einer gewünschten Säure HA dadurch hergestellt, dass man Lösungen der freien Base I mit wenigstens einem Äquivalent der Säure HA behandelt; bevorzugt sind entweder alkoholische Lösungen wie etwa eine methanolische Lösung oder wässrige Lösung. Das Säureadditionssalz kristallisiert entweder sofort oder nach Entfernen des Lösemittels, gegebenenfalls nach Umkristallisation aus einem geeigneten Lösemittel.

Als Säuren HA für pharmazeutisch bevorzugte Säureadditionssalze kommen

organische Säuren wie Weinsäure, Äpfelsäure, Milchsäure, Essigsäure, Citronensäure, Methansulfonsäure, Benzolsulfonsäure u.a. oder

anorganische Säuren, wie Chlorwasserstoff-,

Bromwasserstoff-, Jodwasserstoffsäure, Schwefelsäure, Salpetersäure, Phosphorsäure, Amidosulfonsäure usw. in Frage.

Besonders bevorzugt sind die gut wasserlöslichen Hydrohalogenide der Amine I, wie z.B. das Hydrochlorid.

Erfindungsgemäss können ausser den in den Ausführungsbeispielen beschriebenen Verbindungen auch die in der folgenden Tabelle zusammengestellten Verbindungen der allgemeinen Formel I erhalten werden:

| $R_1$ | $R_2$ | $R_3$ | $R_4$ | $R_5$ | $R_6$ | $R_7$ |
|---|---|---|---|---|---|---|
| H | H | H | t-Amyl | H | H | H |
| H | H | H | n-Pentyl | H | H | H |
| H | H | H | 1-Methyl-cyclohexyl | H | H | H |
| H | H | H | cyclo-$C_6H_{11}$ | H | H | H |
| H | H | H | cyclo-$C_7H_{13}$ | H | H | H |
| H | H | H | 1,1-Dimethyl-butyl | H | H | H |
| H | H | H | 1,1-Diethyl-propyl | H | H | H |
| H | H | $OCH_3$ | Cl | $OCH_3$ | H | H |
| H | H | $OC_2H_5$ | Cl | $OC_2H_5$ | H | H |
| H | H | $OCH_3$ | t-Butyl | $OCH_3$ | H | H |
| H | H | $CH_3$ | $CH_3$ | $CH_3$ | H | H |
| H | H | $CH_3$ | t-Butyl | $CH_3$ | H | H |
| H | H | $CH_3$ | $C_2H_5$ | $CH_3$ | H | H |
| H | H | $C_2H_5$ | $CH_3$ | $C_2H_5$ | H | H |
| $C_3H_7$ | $C_3H_7$ | H | t-Butyl | H | H | H |
| $C_4H_9$ | $C_4H_9$ | H | t-Butyl | H | H | H |
| H | H | $C_2H_5$ | Cl | $C_2H_5$ | H | H |
| H | $C_3H_7$ | H | t-Butyl | H | H | H |
| $(CH_2)_5$ | | H | t-Butyl | H | H | H |
| H | H | H | Cl | F | H | H |
| H | H | H | Cl | Cl | H | H |
| H | H | H | Cl | $CH_3$ | H | H |
| H | H | H | Cl | $OCH_3$ | H | H |
| H | H | F | Cl | H | H | H |
| H | H | Cl | Cl | H | H | H |
| H | H | $CH_3$ | Cl | H | H | H |
| H | H | $OCH_3$ | Cl | H | H | H |
| H | H | $(CH_2)_4$ | | H | H | H |
| H | H | H | $(CH_2)_4$ | | H | H |

Die erfindungsgemässen Verbindungen der Formel I sowie deren pharmazeutisch verträgliche Salze sind Diuretika und Salidiuretika, die als Pharmazeutika in der Human- und Veterinärmedizin eingesetzt werden können. Sie werden in täglichen Dosierungen von 0,5 bis 300 mg, vorzugsweise 5-100 mg, insbesondere 5-50 mg gerechnet für einen normal gewichtigen Erwachsenen in Kapseln, Dragees, Tabletten oder Lösungen mit verschiedenen Zusätzen enteral z.B. oral mit Sonde oder dgl. oder parenteral (Injektionen in das Gefässsystem, z.B. intravenös oder auch Injektion in die Muskulatur oder unter die Haut und dgl.) verabfolgt. Sie eignen sich sowohl zur Behandlung der Hypertonie als auch zur Behandlung von Ödemkrankheiten wie kardial, renal oder hepatisch bedingten Ödemen und anderen auf Störung des Wasser- und Elektrolyt-Haushalts zurückzuführenden Erscheinungen.

Die Verbindungen können allein oder in Kombination mit anderen salidiuretisch wirksamen Substanzen auch anderer Wirkungsart angewendet werden. Insbesondere sind zu nennen: Spironolacton, Triamteren, Amilorid und andere $K^+$-retinierende Verbindungen. Aber auch andere rein blutdrucksenkende Verbindungen kommen als mögliche Kombinationspartner in Frage, z.B. Hydralazin, Clonidin, Reserpin und insbesondere auch beta-blockierende Substanzen wie etwa Metoprolol oder Penbutolol.

Beispiel 1

2-Aminomethyl-4-(1,1-dimethylethyl)-6-sulfamoylphenol-hydrochlorid

a) 3-(1,1-Dimethylethyl)-6-methoxybenzolsulfonsäurechlorid

12,3 g (0,075 Mol) 4-(1,1-Dimethylethyl)anisol gelöst in 30 ml Methylenchlorid tropft man unter Eiskühlung zu 16,5 ml Chlorsulfonsäure gelöst in 20 ml Methylenchlorid. Man rührt das Gemisch 40 Min. und giesst anschliessend auf Eiswasser. Die organische Phase wird abgetrennt, mit Wasser gewaschen, mit $MgSO_4$ getrocknet und eingeengt.

Umkristallisation aus Toluol/Petrolether liefert Kristalle vom Schmp. 75 - 77°C.

b) 3-(1,1-Dimethylethyl)-6-methoxybenzolsulfonsäureamid

25,3 g (0,1 Mol) 3-(1,1-Dimethylethyl)-6-methoxybenzolsulfonsäurechlorid werden in wenig Aceton gelöst und bei Raumtemperatur langsam in 100 ml konz. Ammoniaklösung eingetropft. Man rührt 30 Min. bei Raumtemperatur und giesst auf Eiswasser. Nach Ansäuern mit konz. Salzsäure wird abgesaugt. Die Umkristallisation erfolgt aus Isopropanol.

Weisse Kristalle vom Schmp. 156 - 158°C.

c) 4-(1,1-Dimethylethyl)-2-dimethylaminomethylenaminosulfonylanisol

4,68 g (0,02 Mol) 3-(1,1-Dimethylethyl)-6-methoxybenzolsulfonsäureamid werden in 50 ml Dimethylformamid gelöst und mit 2,5 g (0,022 Mol) Dimethylformamiddimethylacetal versetzt. Man belässt 30 Min. bei Raumtemperatur, giesst auf Eiswasser und saugt ab.

Weisse Kristalle vom Schmp. 134 - 136°C.

d) 4-(1,1-Dimethylethyl)-2-dimethylaminomethylenaminosulfonylphenol

2,98 g (0,01 Mol) 4-(1,1-Dimethylethyl-2-dimethylaminomethylenaminosulfonylanisol werden in 30 ml Methylenchlorid gelöst und mit 2,75 g (0,011 Mol) Bortribromid versetzt. Man rührt 45 Min. bei Raumtemperatur und zerstört überschüssiges Bortribromid vorsichtig durch Zugabe von Methanol. Man entfernt das Lösemittelgemisch im Vakuum und verreibt den Rückstand mit Wasser. Das Produkt fällt in Form leicht gelblicher Kristalle an. Die Umkristallisation geschieht aus Isopropanol.

Schmp. 162 - 164°C.

e) 2-Chlor-N-[5-(1,1-dimethylethyl)-3-dimethylaminomethylenaminosulfonyl-2-hydroxybenzyl]acetamid

3 g (0,01 Mol) 4-(1,1-Dimethylethyl)-2-dimethyl-aminomethylenaminosulfonylphenol werden in 30 ml Schwefelsäure gelöst und mit 1,1 g (0,009 Mol) 2-Chlor-N-hydroxymethylacetamid versetzt. Man rührt die Mischung 30 Min. bei Raumtemperatur und giesst auf Eiswasser. Nach dem Absaugen wird aus Methanol umkristallisiert.

Weisse Kristalle vom Schmp. 166 - 169°C.

f) 2-Aminomethyl-4-(1,1-dimethylethyl)-6-sulfamoylphenolhydrochlorid

3 g (0,008 Mol) 2-Chlor-N[5-(61,1-Dimethylethyl)-3-dimethylaminomethylenaminosulfonyl-2-hydroxybenzyl]acetamid werden in einer Mischung aus 30 ml Ethanol und 3 ml konz. Salzsäure 2 Stunden am Rückfluss erhitzt. Man zieht das Lösemittel im Vakuum ab und kristallisiert den Rückstand aus Methanol/Ether.

Weisse Kristalle vom Schmp. 226 - 228°C (Zers.).

Beispiel 2

2-Aminomethyl-4-isopropyl-6-sulfamoylphenolhydrochlorid

Die Verbindung wird analog der in Beispiel 1 beschriebenen Reaktionsfolge, jedoch mit 4-Isopropylanisol als Ausgangsmaterial hergestellt.

Die Zwischenprodukte und das Endprodukt weisen folgende Schmelzpunkte auf:

a) 3-Isopropyl-6-methoxybenzolsulfonsäurechlorid: Schmp. 60 - 62°C.

b) 3-Isopropyl-6-methoxybenzolsulfonsäureamid: Schmp. 167 -169°C.

c) 2-Dimethylaminomethylenaminosulfonyl-4-isopropylanisol, Schmp. 124 - 125°C.

d) 2-Dimethylaminomethylenaminosulfonyl-4-isopropylphenol, Schmp. 97 - 99°C.

e) 2-Chlor-N-(3-Dimethylaminomethylenaminosulfonyl-2-hydroxy-5-isopropylbenzyl)-acetamid, Schmp. 124 - 126°C.

f) 2-Aminomethyl-4-isopropyl-6-sulfamoylphenolhydrochlorid, Schmp. 243 - 246°C (Zers.).

Beispiel 3

2-Aminomethyl-4-propyl-6-sulfamoylphenolhydrochlorid

Die Verbindung wird analog der in Beispiel 1 beschriebenen Reaktionsfolge, jedoch mit 4-Propylanisol als Ausgangsmaterial hergestellt. Die Zwischenprodukte und das Endprodukt weisen folgende Schmelzpunkte auf:

a) 2-Methoxy-2-propylbenzolsulfonsäurechlorid, Öl.

b) 2-Methoxy-5-propylbenzolsulfonsäureamid, Schmp. 132 - 134°C.

c) 2-Dimethylaminomethylenaminosulfonyl-4-propylanisol, Schmp. 118 - 121°C.

d) 2-Dimethylaminomethylenaminosulfonyl-4-propylphenol, Schmp. 88 - 90°C.

e) 2-Chlor-N-(3-Dimethylaminomethylenaminosulfonyl-2-hydroxy-5-propylbenzyl)-acetamid, Schmp. 111 - 114°C.

f) 2-Aminomethyl-4-propyl-6-sulfamoylphenolhydrochlorid, Schmp. 232 - 235°C (Zers.).

Beispiel 4

2-Aminomethyl-4-methyl-6-sulfamoylphenolhydrochlorid

Die Verbindung wird analog der im Beispiel 1 beschriebenen Reaktionsfolge, jedoch mit 4-Methylanisol als Ausgangsmaterial hergestellt. Die Zwischenprodukte und das Endprodukt weisen folgende Schmelzpunkte auf:

a) 2-Methoxy-5-methylbenzolsulfonsäurechlorid, Schmp. 86 - 88°C.

b) 2-Methoxy-5-methylbenzolsulfonsäureamid, Schmp. 185 - 188°C.

c) 2-Dimethylaminomethylenaminosulfonyl-4-methylanisol, Schmp. 146 - 148°C.

d) 2-Dimethylaminomethylenaminosulfonyl-4-methyl-phenol, Schmp. 150 - 152°C.

e) 2-Chlor-N-(3-Dimethylaminomethylenaminosulfonyl-2-hydroxy-5-methylbenzyl)acetamid, Schmp. 176 -178°C.

f) 2-Aminomethyl-4-methyl-6-sulfamoylphenolhydrochlorid, Schmp. 230 - 233°C (Zers.).

Beispiel 5

2-Aminomethyl-4-chlor-6-sulfamoylphenolhydrochlorid

Die Verbindung wurde analog der im Beispiel 1 beschriebenen Reaktionsfolge, jedoch mit 4-Chloranisol als Ausgangsmaterial hergestellt. Die Zwischenprodukte sowie das Endprodukt weisen folgende Schmelzpunkte auf:

a) 3-Chlor-6-methoxybenzolsulfonsäurechlorid, Schmp. 102 - 103°C.

b) 3-Chlor-6-Methoxybenzolsulfonsäureamid, Schmp. 147 - 149°C.

c) 2-Dimethylaminomethylenaminosulfonyl-4-chloranisol, Schmp. 171 - 174°C.

d) 2-Dimethylaminomethylenaminosulfonyl-4-chlorphenol, Schmp. 159 - 162°C.

e) 2-Chlor-N-(3-Dimethylaminomethylenaminosulfonyl-5-chlorbenzyl)acetamid, Schmp. 135 - 140°C.

f) 2-Aminomethyl-4-chlor-6-sulfamoylphenolhydrochlorid, Schmp. 252 -255°C (Zers.).

Beispiel 6

2-Aminomethyl-4-(1-methylpropyl)-6-sulfamoylphenolhydrochlorid

Die Verbindung wurde analog der in Beispiel 1 beschriebenen Reaktionsfolge, jedoch mit 4-(1-Methylpropyl)-anisol als Ausgangsmaterial hergestellt. Die Zwischenprodukte sowie das Endprodukt wiesen folgende Schmelzpunkte auf:

a) 2-Methoxy-5-(1-methylpropyl)-benzolsulfonsäurechlorid, Schmp: Öl.

b) 2-Methoxy-5-(1-methylpropyl)-benzolsulfonsäureamid, Schmp. 120 -122°C.

c) 2-Dimethylaminomethylenaminosulfonyl-4-(1-methyl-propyl)anisol, Schmp. 93 - 95°C.

d) 2-Dimethylaminomethylenaminosulfonyl-4-(1-methylpropyl)phenol, Schmp. 87 - 91°C.

e) 2-Chlor-N-[3-dimethylaminomethylenaminosulfonyl-2-hydroxy-5-(1-methylpropyl)benzyl]acetamid, Schmp. 103 - 105°C.

f) 2-Aminomethyl-4-(1-methylpropyl)-6-sulfamoylphenol-hydrochlorid, Schmp. 218 - 222°C (Zers.).

Beispiel 7

2-Aminomethyl-4-ethyl-6-sulfamoylphenolhydrochlorid

Die Verbindung wird analog der in Beispiel 1 beschriebenen Reaktionsfolge, jedoch mit 4-Ethylanisol als Ausgangsmaterial hergestellt. Die Zwischenprodukte sowie das Endprodukt wiesen folgende Schmelzpunkte auf:

a) 2-Methoxy-5-ethylbenzolsulfochlorid, Schmp. 58 -60°C.

b) 2-Methoxy-5-ethylbenzolsulfonsäureamid, Schmp. 162 - 165°C.

c) 2-Dimethylaminomethylenaminosulfonyl-4-ethylanisol, Schmp. 140 - 142°C.

d) 2-Dimethylaminomethylenaminosulfonyl-4-ethylphenol, Schmp. 100 - 102°C.

e) 2-Chlor-N-(3-dimethylaminomethylenaminosulfonyl-2-hydroxy-5-ethylbenzyl)acetamid, Schmp. 130 - 133°C.

f) 2-Aminomethyl-4-ethyl-6-sulfamoylphenol-hydrochlorid, Schmp. 234 - 238°C (Zers.).

Beispiel 8

2-Aminomethyl-4-chlor-3,5-dimethyl-6-sulfamoylphenolhydrochlorid

Die Verbindung wurde analog der in Beispiel 1 beschriebenen Reaktionsfolge, jedoch mit 4-Chlor-3,5-dimethylphenol als Ausgangsmaterial hergestellt. Die Zwischenprodukte sowie das Endprodukt wiesen folgende Schmelzpunkte auf:

a) 3-Chlor-2,4-dimethyl-6-methoxybenzolsulfonsäurechlorid Schmp. 88 - 90°C.

b) 3-Chlor-2,4-dimethyl-6-methoxybenzolsulfonsäureamid, Schmp. 208 - 212°C.

c) 2-Dimethylaminomethylenaminosulfonyl-4-chlor-3,5-dimethylanisol, Schmp. 204 -206°C.

d) 2-Dimethylaminomethylenaminosulfonyl-4-chlor-3,5-dimethylphenol, Schmp. 142 - 146°C.

e) 2-Chlor-N-(3-dimethylaminomethylenaminosulfonyl-5-chlor-4,6-dimethyl-2-hydroxybenzyl)acetamid, Schmp. 130 - 133°C.

f) 2-Aminomethyl-4-chlor-3,5-dimethyl-6-sulfamoylphenol, hydrochlorid, Schmp. 256 - 260°C (Zers.).

Beispiel 9

1-Aminomethyl-3-sulfamoyl-5,6,7,8-tetrahydro-2-naphtolhydrochlorid

a) 3-Chlorsulfonyl-5,6,7,8-tetrahydro-2-naphtolmethylether

118 g (0,73 Mol) 5,6,7,8-Tetrahydro-2-naphtolmethylether werden in 320 ml Methylenchlorid gelöst, und mit 177 ml Chloroschwefelsäure in 160 ml Methylenchlorid versetzt. Man rührt 10 Min. bei 5°C und gibt auf Eiswasser. Die org. Phase wird abgetrennt, mit $MgSO_4$ getrocknet. Das Lösungsmittel wird abgedampft und der Rückstand aus Aceton/Petrolether umkristallisiert.

Weisse Kristalle vom Schmp. 103 - 104°C.

b) 3-Dimethylaminomethylenaminosulfonyl-5,6,7,8-tretrahydro-2-naphtolmethylether

32 g (0,013 Mol) 3-Chlorsulfonyl-5,6,7,8-tretrahydro-2-naphtolmethylether werden in wenig Aceton gelöst, die Lösung in 100 ml konz. Ammoniaklösung eingetropft und 30 Min. bei Raumtemperatur belassen. Man säuert mit konz. Salzsäure an, schüttelt mit Essigsäureethylester aus, trocknet mit $MgSO_4$ und engt ein. Den Rückstand löst man in 100 ml Dimethylformamid und gibt 24 g (0,2 Mol) Dimethylformamidmethylacetal dazu. Man rührt 30 Min. bei RT, gibt auf Eiswasser und saugt ab. Die Umkristallisation aus Methanol liefert Kristalle vom Schmp. 210 -213°C.

c) 3-Dimethylaminomethylenaminosulfonyl-5,6,7,8-tetrahydro-2-naptol

Die Etherspaltung wird analog Beispiel 1d durchgeführt.

Weisse Kristalle vom Schmp. 163 - 164°C.

d) 2-Chlor-N-(3-dimethylaminomethylenaminosulfonyl-2-hydroxy-5,6,7,8-tetrahydronaphthylmethyl)acetamid

Diese Verbindung wird analog Beispiel 1e hergestellt.

Schmp. 99 - 101°C.

f) 1-Aminomethyl-3-sulfamoyl-5,6,7,8-tetrahydro-2-naphtolhydrochlorid

Diese Verbindung wird analog Beispiel 1f dargestellt. Schmp. 240 -244°C (Zers.)

Beispiel 10

2-Aminomethyl-4-cyclopentyl-6-sulfamoylphenolhydrochlorid

a) 1-Acetamidomethyl-2-acetoxy-4-cyclopentylbenzol

15 g (0,07 Mol) 2-Aminomethyl-4-cyclopentylphenol-hydrochlorid werden in 150 ml Dioxan suspendiert. Man tropft 12 g Pyridin (0,15 Mol) sowie 11,8 g (0,15 Mol) Acetylchlorid zu und rührt 2,5 Stunden bei 90°C. Man giesst auf Eiswasser, saugt ab und trocknet an der Luft.

Weisse Kristalle vom Schmp. 203 - 205°C.

b) Acetamidomethyl-4-cyclopentyl-2-hydroxy-benzolsulfonsäureamid

11 g (0,04 Mol) 1-Acetamidomethyl-2-acetoxy-4-cyclopentylbenzol werden bei RT in 100 ml Chlorschwefelsäure eingetragen. Man rührt 15 Min. bei Raumtemperatur und giesst auf Eiswasser. Man saugt ab, löst den Rückstand in Essigsäuremethylester, trocknet mit $MgSO_4$ und chromatographiert die Lösung als Kieselgel, wobei mit Ethylacetat eluiert wird. Man zieht das Lösungsmittel im Vakuum ab, löst den Rückstand in wenig Aceton und tropft ihn in 100 ml konz. Ammoniaklösung ein. Man rührt 30 Min. bei Raumtemperatur und säuert mit konz. Salzsäure auf pH 2 an.

Man saugt ab, wäscht mit viel Wasser nach und verkocht den erhaltenen Kristallbrei mit n-Butanol.

Weisse Kristalle vom Schmp. 243 - 246°C.

c) 2-Aminomethyl-4-cyclopentyl-6-sulfamoyl-phenol-hydrochlorid

3,4 g (0,012 Mol) 3-Acetamidomethyl-2-acetoxy-2-hydroxy-benzolsulfonsäureamid werden in einer Mischung aus 30 ml Ethanol und 10 ml konz. Salzsäure 8 Stunden am Rückfluss erhitzt. Nach Abziehen des Lösungsmittels wird der Rückstand aus Methanol/Ether umkristallisiert.

Weisse Kristalle vom Schmp. 243 - 246°C.

Beispiel 11

2-Aminomethyl-4-(1,1-dimethylethyl)-6-methylsulfamoyl-phenolhydrochlorid

a) 2-Acetamidomethyl-4(1,1-dimethylethyl) phenol

43 g (0,2 Mol) 2-Aminomethyl-4(1,1-dimethylethyl)-phenolhydrochlorid werden in 400 ml 1,4-Dioxan suspendiert und mit 40 ml Pyridin versetzt. Unter Rühren und Eiskühlung tropft man 40 ml (0,55 Mol) Acetylchlorid zu. Man rührt anschliessend 2,5 Stunden bei 90°C, gibt auf Eiswasser und saugt den auskristallisierten Niederschlag ab.

Weisse Kristalle vom Schmp. 99 - 102°C.

b) 2-Acetamidomethyl-6-chlorsulfonyl-4-(1,1-dimethylethyl)phenol

15,5 g (0,059 Mol) 2-Acetamidomethyl-4(1,1-dimethylethyl)phenol werden bei Raumtemperatur in 100 ml Chloroschwefelsäure eingetragen. Man rührt 15 Min. nach und rührt die Lösung in Eiswasser ein. Der abgesaugte Niederschlag wird aus Toluol umkristallisiert.

Farblose Kristalle vom Schmp. 145 - 147°C.

c) 2-Acetamidomethyl-4-(1,1-dimethylethyl)6-methylsulfamoylphenol

9 g (0,028 Mol) 2-Acetamidomethyl-6-chlorsulfonyl-4-(1,1-dimethylethyl)phenol werden in 100 ml Aceton gelöst, unter Eiskühlung werden 4,9 ml (0,056 Mol) 40% wässrige Methylaminlösung zugetropft. Man rührt 30 Min. bei Raumtemperatur und giesst auf Eiswasser. Die Umkristallisation aus n-Propanol liefert weisse Kristalle vom Schmp. 173 - 174°C.

d) 2-Aminomethyl-4(1,1-dimethylethyl)-6-methylsulfamoylphenolhydrochlorid

4,9 g (0,016 Mol) 6-Acetamidomethyl-4-(1,1-dimethylethyl-2-methylsulfamoylphenol werden in einer Mischung aus 30 ml Ethanol und 10 ml konz. Salzsäure 8 Stunden am Rückfluss erhitzt. Eindampfen und Umkristallisation aus Methanol/Ether liefert weisse Kristalle vom Schmp. 238 - 240°C.

Beispiel 12

2-Aminomethyl-4-(1,1-dimethylethyl)-6-dimethylsulfamoylphenolhydrochlorid

Diese Verbindung wird aus 2-Acetamidomethyl-6-chlorsulfonyl-4-(1,1-dimethylethyl)phenol und Dimethylamin analog der Beispiele 11c und 11d hergestellt.

Weise Kristalle vom Schmp. 129–131°C.

Beispiel 13

2-Aminomethyl-6-diethylsulfamoyl-4-(1,1-dimethylethyl)phenolhydrochlorid

Diese Verbindung wird analog der Beispiele 11c und 11d aus 2-Acetamidomethyl-6-chlorsulfonyl-4-(1,1-dimethylethyl)phenol und Diäthylamin hergestellt.

Farblose Nadeln vom Schmp. 125 - 127°C.

Beispiel 14

2-Aminomethyl-4-(1,1-dimethylethyl)6-ethylsulfamoylphenolhydrochlorid

Diese Verbindung wird analog der Beispiele 11c und 11d aus 2-Acetamidomethyl-6-chlorsulfonyl-4-(1,1-dimethylethyl)phenol und Ethylamin hergestellt.

Weisse Kristalle vom Schmp. 102 - 105°C.

Beispiel 15

2-Aminomethyl-6-butylsulfamoyl-4-(1,1-dimethylethyl)phenolhydrochlorid

Diese Verbindung wird analog der Beispiele 11c und 11d aus 2-Acetamidomethyl-6-chlorsulfonyl-4-(1,1-dimethylethyl)phenol und Butylamin hergestellt.

Weisse Kristalle vom Schmp. 81 - 84°C.

Beispiel 16

2-Aminomethyl-4-(1,1-dimethylethyl)-6(1-pyrrolidinylsulfonyl)phenolhydrochlorid

Diese Verbindung wird analog der Beispiele 11c und 11d aus 2-Acetamidomethyl-6-chlorsulfonyl-4-(1,1-dimethylethyl)phenol und Pyrrolidin hergestellt.

Farblose Kristalle vom Schmp. 168 - 172°C.

Beispiel 17

4-Aminomethyl-5-hydroxy-6-sulfamoylindan-hydrochlorid

a) 6-Chlorsulfonyl-5-methoxyindan

50 g (0,34 Mol) 5-Methoxyindan werden analog Beispiel 1a mit 75 ml Chlorsulfonsäure zum

6-Chlorsulfonyl-5-methoxyindan umgesetzt.
Weisse Kristalle vom Schmp. 75 - 77°C.

b) 5-Methoxy-6-sulfamoylindan
Diese Verbindung wird analog Beispiel 1b aus 6-Chlorsulfonyl-5-methoxyindan hergestellt.
Weisse Kristalle vom Schmp. 91 - 92°C.

c) 5-Methoxy-6-dimethylaminomethylenaminosulfonylindan
Diese Verbindung wird analog Beispiel 1c aus 5-Methoxy-6-sulfamoylindan und Dimethylformamid-dimethylacetal hergestellt.
Weisse Kristalle vom Schmp. 238 - 241°C.

d) 5-Hydroxy-6-dimethylaminomethylenaminosulfonylindan
Diese Verbindung wird analog Beispiel 1d aus 5-Methoxy-6-dimethylaminomethylenaminosulfonylindan und Bortribromid hergestellt.
Weisse Kristalle vom Schmp. 180 - 182°C.

e) 4-(N-Chloracetylaminomethyl)-5-hydroxy-6-dimethyl-aminomethylenaminosulfonylindan
Diese Verbindung wird analog Beispiel 1e aus 5-Hydroxy-6-dimethylaminomethylenaminosulfonylindan hergestellt.
Weisse Kristalle vom Schmp. 156 - 158°C.

f) 4-Aminomethyl-5-hydroxy-6-sulfamoylindanhydrochlorid
Die Verbindung wird analog Beispiel 1f aus 4-(N-Chloracetylaminomethyl)-5-hydroxy-6-dimethylaminomethylenaminosulfonylindan hergestellt.
Weisse Kristalle vom Schmp. 256 - 258°C (Zers.).

Beispiel 18

6-Aminomethyl-5-hydroxy-4-sulfamoylindanhydrochlorid

a) 6-(N-Chloracetylaminomethyl)-5-hydroxyindan
134 g (1 Mol) 5-Hydroxyindan und 100 g 2-Chlor-N-hydroxymethylacetamid (0,81 Mol) werden in 300 ml Eisessig suspendiert und mit 10 ml konz. Schwefelsäure versetzt. Man rührt 30 Min. bei Raumtemperatur und giesst auf Eiswasser. Man saugt ab und kristallisiert aus Methanol um.
Farblose Kristalle vom Schmp. 158 - 161°C.

b) 6-(N-Chloracetylaminomethyl)-5-hydroxy-4-chlorsulfonylindan
70 g (0,29 Mol) 6-(N-Chloracetylaminomethyl)-5-hydroxyindan werden in 150 ml Chlorsulfonsäure gelöst und ca. 30 Min. bei Raumtemperatur gerührt. Man giesst auf Eiswasser und saugt ab. Das zunächst kristalline Material wird nach einiger Zeit ölig und in dieser Form ohne weitere Reinigung in der nächsten Stufe eingesetzt.

c) 6-(N-Chloracetylaminomethyl)-5-hydroxy-4-sulfamoylindan
33,8 g (0,1 Mol) 6-(N-Chloracetylaminomethyl)-5-hydroxy-4-chlorsulfonylindan werden in wenig Aceton gelöst und zu 50 ml konz. Ammoniaklösung getropft. Man rührt 30 Min. bei Raumtemperatur und verdünnt mit Wasser. Der ausgefällte Niederschlag wird abgesaugt, in Aceton gelöst und nach Filtration erneut in Wasser eingerührt.
Weisse Kristalle vom Schmp. 209 - 212°C.

d) 6-Aminomethyl-5-hydroxy-4-sulfamoylindandhydrochlorid
Diese Verbindung wird analog Beispiel 1f aus 6-(N-Chloracetylaminomethyl)-5-hydroxy-4-sulfamoylindan hergestellt.
Weisse Kristalle vom Schmp. 245 - 248°C (Zers.).

Beispiel 19

2-Aminomethyl-4-(1-methylcyclohexyl)-6-sulfamoylphenolhydrochlorid
Diese Verbindung wurde analog der in Beispiel 1 beschriebenen Reaktionsfolge, jedoch mit 4-(1-Methylcyclohexyl)anisol als Ausgangsmaterial hergestellt. Die Zwischenprodukte sowie das Endprodukt wiesen folgende Schmelzpunkte auf:
a) 2-Methoxy-5-(1-methylcyclohexyl)benzolsulfonsäurechlorid, Schmp. Öl.
b) 2-Methoxy-5-(1-methylcyclohexyl)benzolsulfonsäureamid, Schmp. 148 - 151°C.
c) 2-Dimethylaminomethylenaminosulfonyl-4-(1-methylcyclohexyl)anisol, Schmp. 78 - 80°C.
d) 2-Dimethylaminomethylenaminosulfonyl-4-(1-methylcyclohexyl)phenol, Schmp. 120 - 22°C.
e) 2-Chlor-N-[3-dimethylaminomethylenaminosulfonyl-2-hydroxy-5-(1-methylcyclohexyl) benzyl]acetamid, Schmp. 123 - 25°C.
f) 2-aminomethyl-4-(1-methylcyclohexyl)-6-sulfamoylphenolhydrochlorid, Schmp. 193 - 197°C.

Beispiel 20

2-Aminomethyl-4-(1,1-dimethylethyl)-6-(o-chlorbenzyl)-aminosulfonylphenolhydrochlorid
Diese Verbindung wird aus 2-Acetamidomethyl-6-chlorsulfonyl-4-(1,1-dimethylethyl)phenol und o-Chlorbenzylamin analog der Beispiele 11c und 11d hergestellt. Schmp. 180 - 183°C.

Beispiel 21

2-Aminomethyl-4-(1,1-dimethylethyl)-6-alkylaminosulfonyl-phenolhydrochlorid
Analog Beispiel 11c und 11d aus Alkylamin und 2-Acetamidomethyl-6-chlorsulfonyl-4-(1,1-dimethylethyl)phenol Schmp. 140-43°C

Beispiel 22

2-Dimethylaminomethyl-4-(1,1-dimethylethyl)-6-sulfamoylphenolhydrochlorid
2 g (0,007 Mol) 2-Dimethylaminomethylenaminosulfonyl-4-(1,1-dimethylethyl)phenol werden in 11 ml Ethanol gelöst, mit 3,7 ml 35% Formalinlösung sowie mit 4,7 ml 40% Dimethylaminlösung in Wasser versetzt und 1 Stunde am Rückfluss gekocht. Die Lösung wird bis zur Trockene eingeengt, der Rückstand mit 2 N Salzsäure aufgenommen und mehrfach mit Ethylacetat extrahiert. Die wässrige Phase wird mit Ethylacetat extrahiert. Die wässrige Phase wird erneut eingeengt, mit Ethanol und wenig konz. Salzsäure gelöst, ca. 20 Min. am Rückfluss gekocht und nachfolgend bis zur beginnenden Kristallisation eingeengt.

Weisse Kristalle vom Schmp. 210–213°C

Beispiel 23

2-Aminomethyl-4-(1,1-dimethylpropyl)-6-sulfamoylphenolhydrochlorid

Diese Verbindung wurde analog der in Beispiel 1 beschriebenen Reaktionsfolge, jedoch mit 4-(1,1-Dimethylpropyl)anisol als Ausgangsmaterial hergestellt. Die Zwischenprodukte sowie das Endprodukt zeigte folgende Schmelzpunkte:

a) 2-Methoxy-5-(1,1-dimethylpropyl)benzolsulfonsäurechlorid: Öl.

b) 2-Methoxy-5-(1,1-dimethylpropyl)benzolsulfonsäureamid, Schmp. 117 - 119°C.

c) 2-Dimethylaminomethylenaminosulfonyl-4-(1,1-dimethylpropyl)anisol, Schmp 109 - 111°C.

d) 2-Dimethylaminomethylenaminosulfonyl-4-(1,1-dimethylpropyl)phenol, Schmp. 150 - 152°C.

e) 2-Chlor-N-[3-dimethylaminomethylenaminosulfonyl-2-hydroxy-5-(1,1-dimethylpropyl)benzyl]acetamid, Schmp. 127 - 129°C.

f) 2-Aminomethyl-4-(1,1-dimethylpropyl)-6-sulfamoyl-phenolhydrochlorid, Schmp. 180 - 182°C.

**Patentansprüche für die Vertragsstaaten: BE, CH, DE, FR, GB, IT, LI, NL**

1. 2-Aminomethylphenole der Formel I

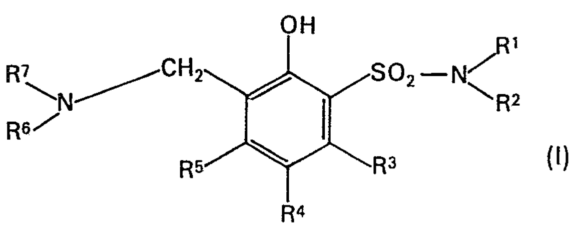

(I)

in welcher

$R^1$ und $R^2$ gleich oder verschieden sind und für Wasserstoff, Alkyl mit 1 bis 8 C-Atomen, Alkenyl mit 2 bis 8 C-Atomen, Alkinyl mit 2 bis 8 C-Atomen, Cycloalkyl mit 3 bis 12 C-Atomen und bis zu 8 Ringgliedern oder Benzyl, das gegebenenfalls durch Alkyl mit 1 bis 4 C-Atomen, Alkoxy mit 1 bis 4 C-Atomen oder Halogen substituiert ist, stehen,

$R^3$ und $R^5$ gleich oder verschieden sind und Wasserstoff, Halogen, Alkyl mit 1 oder 2 C-Atomen oder Alkoxy mit 1 oder 2 C-Atomen,

$R^4$ Halogen, Alkyl mit 1 bis 12 C-Atomen oder Cycloalkyl mit 3 bis 12 C-Atomen und bis zu 8 Ringgliedern bedeuten und

$R^6$ und $R^7$ gleich oder verschieden sind und für Wasserstoff oder Alkyl mit 1 bis 4 C-Atomen stehen,

wobei die Reste $R^1$ und $R^2$, $R^6$ und $R^7$ und/oder zwei der Reste $R^3$, $R^4$ und $R^5$ eine $-[CH_2]_m-$Kette mit m = 3 bis 6 bilden können, die gegebenenfalls durch 1 oder 2 Methylgruppen substituiert und die im Falle der Reste $R^1$, $R^2$, $R^6$ und $R^7$ auch durch 1 oder 2 Sauerstoffatome, Schwefelatome und/oder Iminogruppen unterbrochen sein kann, sowie deren physiologisch verträglichen Salze.

2. Verbindungen der Formel I gemäss Anspruch 1, dadurch gekennzeichnet, dass

$R^1$ und $R^2$ gleich oder verschieden sind und für Wasserstoff, Alkyl mit 1 bis 4 C-Atomen, Alkenyl oder Alkinyl mit 2 bis 4 C-Atomen und Benzyl, das gegebenenfalls durch Methyl, Methoxy oder Halogen substituiert ist, stehen,

$R^3$ und $R^5$ die im Anspruch 1 genannte Bedeutung haben,

$R^4$ Halogen, Alkyl mit 1 bis 8 C-Atomen, Cycloalkyl mit 3 bis 10 C-Atomen und bis 7 Ringgliedern und

$R^6$ und $R^7$ gleich oder verschieden sind und Wasserstoff oder Alkyl mit 1 oder 2 C-Atomen bedeuten,

wobei die Reste $R^1$ und $R^2$, $R^6$ und $R^7$ und/oder zwei der Reste $R^3$, $R^4$ und $R^5$ eine im Anspruch 1 definierte Polymethylenkette bilden können.

3. 2-Aminomethyl-4-(1,1-dimethylethyl)-6-sulfamoylphenol sowie dessen physiologisch verträgliche Säureadditionssalze.

4. 2-Aminomethyl-4-isopropyl-6-sulfamoylphenol sowie dessen physiologisch verträgliche Säureadditionssalze.

5. 2-Aminomethyl-4-(1-methylpropyl)-6-sulfamoyl-phenol sowie dessen physiologisch verträgliche Salze.

6. 2-Aminomethyl-4-(1,1-dimethylethyl)-6-N-pyrrolidinylsulfonylphenol sowie dessen physiologisch verträgliche Säureadditionssalze.

7. Verfahren zur Herstellung von 2-Aminomethylphenolen gemäss Anspruch 1, dadurch gekennzeichnet, dass man

a) ein Phenol der Formel II

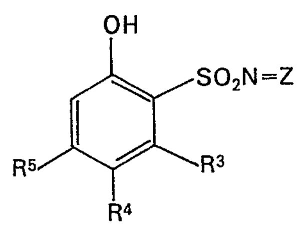

(II)

worin die Reste $R^3$ bis $R^5$ die im Anspruch 1 genannten Bedeutungen haben und Z entweder für die beiden Reste $R^1$ und $R^2$, die die im Anspruch 1 genannte Bedeutungen mit Ausnahme der des Wasserstoffs haben oder für eine Sulfonamidschutzgruppe der Formel III

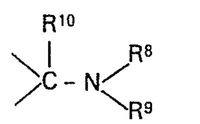

(III)

in der die Reste $R^{10}$ Wasserstoff oder Alkyl mit 1 bis 4 C-Atomen und $R^8$ und $R^9$ Alkyl mit 1 bis 4 C-Atomen bedeuten, stehen, mit einem N-Hydroxymethylcarboxamid der Fomel IV,

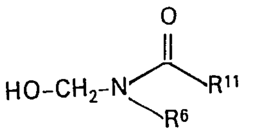

(IV)

in der $R^6$ die im Anspruch 1 definierte Bedeutung hat und der Rest CO-$R^{11}$ für eine Acylgruppe, wobei $R^{11}$ Wasserstoff, gegebenenfalls halogensubstituiertes Alkyl mit 1 bis 4 C-Atomen oder Aryl mit 6 bis 10 C-Atomen sein kann, steht oder in der $R^6$ und der Rest CO-$R^{11}$ gemeinsam den o-Phtaloylrest bilden, zu Verbindungen der Formel V umsetzt

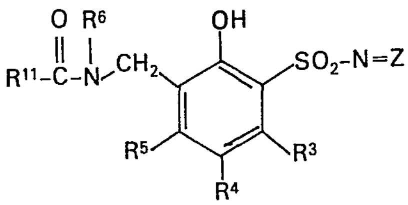

(V)

und den Acylrest CO-$R^{11}$ sowie gegebenenfalls die Sulfonamidschutzgruppe Z hydrolytisch abspaltet,

b) ein Phenol der Formel II, in der die Reste $R^3$, $R^4$ und $R^5$ sowie z die in Anspruch 7a genannten Bedeutungen haben, mit einem Amin der Formel VI

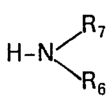

(VI)

in der $R^7$ und $R^6$ die im Anspruch 1 genannte Bedeutung mit Ausnahme der von Wasserstoff besitzen, in Gegenwart von Formaldehyd umsetzt und gegebenenfalls die Sulfonamidschutzgruppe Z hydrolytisch abspaltet,

c) Phenole der Formel VII

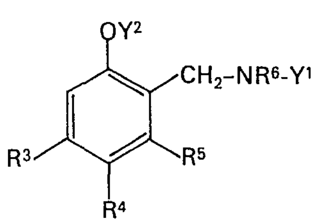

(VII)

in der $R^3$ bis $R^6$ die im Anspruch 1 genannten Bedeutungen haben und $Y^1$ für den Rest $R^{11}$ -CO- steht, wobei $R^{11}$ die oben genannte Bedeutung hat und $Y^2$ entweder Wasserstoff oder $Y^1$ bedeutet, chlorsulfoniert zu Verbindungen der Formel IX

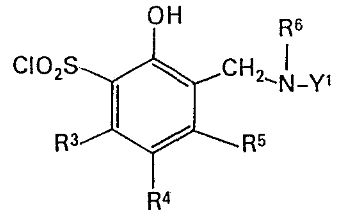

(IX)

und diese mit Aminen der Formel X

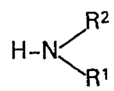

(X)

in der $R^1$ und $R^2$ die in Anspruch 1 erwähnten Bedeutungen haben, zu Verbindungen der Formel XI umsetzt

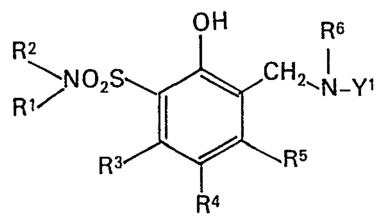

(XI)

und aus diesen den Rest $Y^1$ hydrolytisch abspaltet oder

d) Verbindungen der Formel XVIII

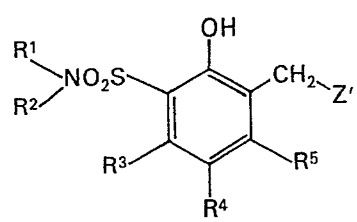

(XVIII)

in der $R^1$ bis $R^5$ die in Anspruch 1 erwähnten Bedeutungen haben, wobei $R^1$ und $R^2$ aber auch gemeinsam für die bei Verfahrensvariante a erwähnte Sulfonamidschutzgruppe Z stehen können und Z' für eine Fluchtgruppe steht, mit Aminen der Formel H-N = Y', wobei Y' entweder für die oben definierten Reste $R^6$ oder $R^7$ oder für einen der Reste $R^6$ oder $R^7$ und eine Aminschutzgruppe steht oder Y' allein eine Aminschutzgruppe bedeutet zu Verbindungen der Formel XIX umsetzt

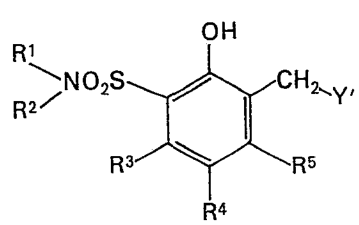

(XIX)

und aus diesen gegebenenfalls die Aminschutzgruppe und/oder die Sulfonamidschutzgruppe hydrolytisch oder hydrogenolytisch abspaltet.

8. Pharmazeutische Präparate auf Basis einer Verbindung gemäss Anspruch 1.

9. Verfahren zur Herstellung eines pharmazeutischen Präparats gemäss Anspruch 8, dadurch gekennzeichnet, dass man eine Verbindung gemäss Anspruch 1, gegebenenfalls mit pharmakologisch verträglichen Trägern und/oder Stabilisatoren, in eine geeignete Darreichungsform überführt.

10. Verbindungen gemäss Anspruch 1 zur Anwendung in der Behandlung von Ödemkrankheiten und der Hypertonie.

11. Verbindungen der Formel II

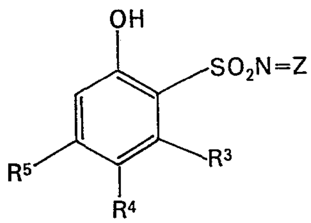

(II)

worin $R^3$ bis $R^5$ die in Anspruch 1 angegebenen Bedeutungen haben und Z für die gemäss Anspruch 7a definierte Sulfonamidschutzgruppe steht.

**Patentansprüche für den Vertragsstaat Österreich:**

1. Verfahren zur Herstellung von 2-Aminomethylphenolen der Formel I

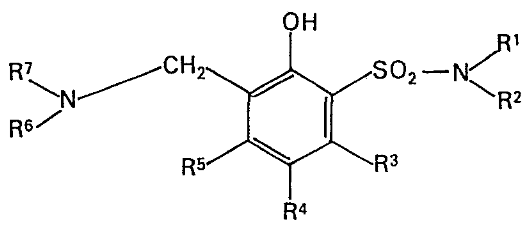

(I)

in welcher
$R^1$ und $R^2$ gleich oder verschieden sind und für Wasserstoff, Alkyl mit 1 bis 8 C-Atomen, Alkenyl mit 2 bis 8 C-Atomen, Alkinyl mit 2 bis 8 C-Atomen, Cycloalkyl mit 3 bis 12 C-Atomen und bis zu 8 Ringgliedern oder Benzyl, das gegebenenfalls durch Alkyl mit 1 bis 4 C-Atomen, Alkoxy mit 1 bis 4 C-Atomen oder Halogen substituiert ist, steht,
$R^3$ und $R^5$ gleich oder verschieden sind und Wasserstoff, Halogen, Alkyl mit 1 oder 2 C-Atomen oder Alkoxy mit 1 oder 2 C-Atomen,
$R^4$ Halogen, Alkyl mit 1 bis 12 C-Atomen oder Cycloalkyl mit 3 bis 12 C-Atomen und bis zu 8 Ringgliedern bedeuten und
$R^6$ und $R^7$ gleich oder verschieden sind und für Wasserstoff oder Alkyl mit 1 bis 4 C-Atomen stehen,
wobei die Reste $R^1$ und $R^2$, $R^6$ und $R^7$ und/oder zwei der Reste $R^3$, $R^4$ und $R^5$ eine $-[CH_2]_m-$Kette mit m = 3 bis 6 bilden können, die gegebenenfalls durch 1 oder 2 Methylgruppen substituiert und die im Falle der Reste $R^1$, $R^2$, $R^6$ und $R^7$ auch durch 1 oder 2 Sauerstoffatome, Schwefelatome und/oder Iminogruppen unterbrochen sein kann, sowie deren physiologisch verträglichen Salzen, dadurch gekennzeichnet, dass man

a) ein Phenol der Formel II

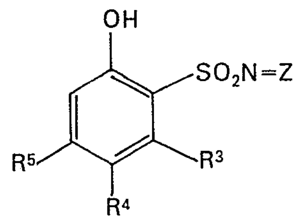

(II)

worin die Reste $R^3$ bis $R^5$ die oben genannten Bedeutungen haben und Z entweder für die beiden Reste $R^1$ und $R^2$, die die oben genannten Bedeutungen mit Ausnahme der des Wasserstoffs haben oder für eine Sulfonamidschutzgruppe der Formel III

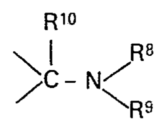

(III)

in der die Reste $R^{10}$ Wasserstoff oder Alkyl mit 1 bis 4 C-Atomen und $R^8$ und $R^9$ Alkyl mit 1 bis 4 C-Atomen bedeuten, stehen, mit einem N-Hydroxymethylcarboxamid der Formel IV,

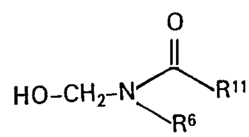

(IV)

in der $R^6$ die oben definierte Bedeutung hat und der Rest $CO-R^{11}$ für eine Acylgruppe, wobei $R^{11}$ Wasserstoff, gegebenenfalls halogen-substituiertes Alkyl mit 1 bis 4 C-Atomen oder Aryl mit 6 bis 10 C-Atomen sein kann, steht oder in der $R^6$ und der Rest $CO-R^{11}$ gemeinsam den o-Phtaloxylrest bilden, zu Verbindungen der Formel V umsetzt

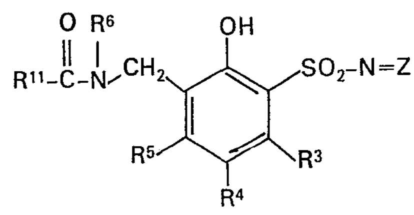

(V)

und den Acylrest $CO-R^{11}$ sowie gegebenenfalls die Sulfonamidschutzgruppe Z hydrolytisch abspaltet,

b) ein Phenol der Formel II, in der die Reste $R^3$, $R^4$ und $R^5$ sowie Z die in Variante a genannten Bedeutungen haben, mit einem Amin der Formel VI

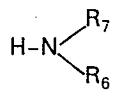

(VI)

in der $R^7$ und $R^6$ die oben genannte Bedeutung mit Ausnahme der von Wasserstoff besitzen, in Gegenwart von Formaldehyd umsetzt und gegebenenfalls die Sulfonamidschutzgruppe Z hydrolytisch abspaltet,

c) Phenole der Formel VII

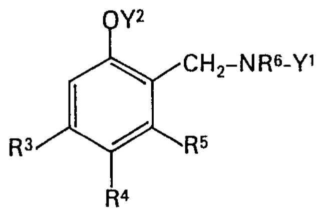

(VII)

in der $R^3$ bis $R^6$ die oben genannten Bedeutungen haben und $Y^1$ für den Rest $R^{11}$ -CO- steht, wobei $R^{11}$ die oben genannte Bedeutung hat und $Y^2$ entweder Wasserstoff oder $Y^1$ bedeutet, chlorsulfiniert zu Verbindungen der Formel IX

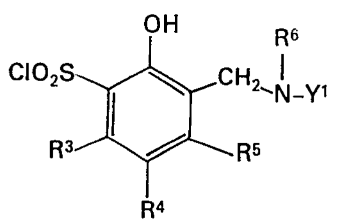

(IX)

und diese mit Aminen der Formel X

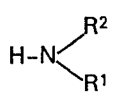

(X)

in der $R^1$ und $R^2$ die oben erwähnten Bedeutungen haben, zu Verbindungen der Formel XI umsetzt

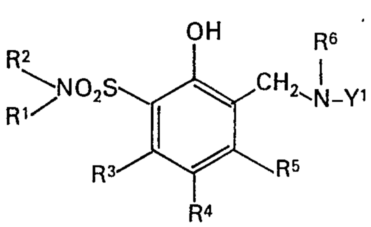

(XI)

und aus diesen den Rest $Y^1$ hydrolytisch abspaltet oder

d) Verbindungen der Formel XVIII

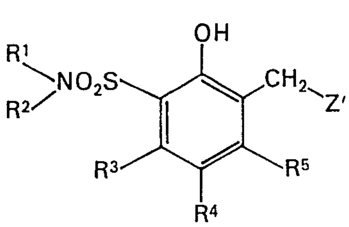

(XVIII)

in der $R^1$ bis $R^5$ die oben erwähnten Bedeutungen haben, wobei $R^1$ und $R^2$ aber auch gemeinsam für die bei Verfahrensvariante a erwähnte Sulfonamidschutzgruppe Z stehen können und $Z'$ für eine Fluchtgruppe steht, mit Aminen der Formel H-N = $Y'$, wobei $Y'$ entweder für die oben definierten Reste $R^6$ und $R^7$ oder für einen der Reste $R^6$ oder $R^7$ und eine Aminschutzgruppe steht oder $Y^1$ allein eine Aminschutzgruppe bedeutet zu Verbindungen der Formel XIX umsetzt

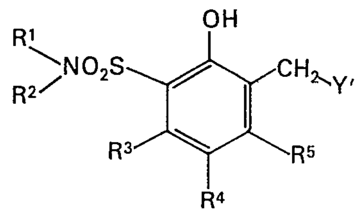

(XIX)

und aus diesen gegebenenfalls die Aminschutzgruppe und/oder die Sulfonamidschutzgruppe hydrolytisch oder hydrogenolytisch abspaltet und die erhaltenen Verbindungen der Formel I gegebenenfalls in ihre Salze überführt.

2. Verfahren gemäss Anspruch 1, dadurch gekennzeichnet, dass Verbindungen der Formel I hergestellt werden, in welcher
$R^1$ und $R^2$ gleich oder verschieden sind und für Waserstoff, Alkyl mit 1 bis 4 C-Atomen, Alkenyl oder Alkinyl mit 2 bis 4 C-Atomen und Benzyl, das gegebenenfalls durch Methyl, Methoxy oder Halogen substituiert ist, steht,
$R^3$ und $R^5$ die im Anspruch 1 genannte Bedeutung haben,
$R^4$ Halogen, Alkyl mit 1 bis 8 C-Atomen, Cycloalkyl mit 3 bis 10 C-Atomen und bis 7 Ringgliedern und
$R^6$ und $R^7$ gleich oder verschieden sind und Wasserstoff oder Alkyl mit 1 oder 2 C-Atomen bedeuten,
wobei die Reste $R^1$ und $R^2$, $R^6$ und $R^7$ und/oder zwei der Reste $R^3$, $R^4$ und $R^5$ eine im Anspruch 1 definierte Polymethylenkette bilden können.

3. Verfahren gemäss Anspruch 1, dadurch gekennzeichnet, dass 2-Aminomethyl-4-(1,1-dimethylethyl)-6-sulfamoylphenol hergestellt und dieses gegebenenfalls in ein physiologisch verträgliches Säureadditionssalz überführt wird.

4. Verfahren gemäss Anspruch 1, dadurch gekennzeichnet, dass 2-Aminomethyl-4-isopropyl-6-sulfamoylphenol hergestellt und dieses gegebenenfalls in ein physiologisch verträgliches Säureadditionssalz überführt wird.

5. Verfahren gemäss Anspruch 1, dadurch gekennzeichnet, dass 2-Aminomethyl-4-(1-methylpropyl)-6-sulfamoylphenol hergestellt und dieses gegebenenfalls in ein physiologisch verträgliches Salz überführt wird.

6. Verfahren gemäss Anspruch 1, dadurch gekennzeichnet, dass 2-Aminomethyl-4-(1,1-dimethylethyl)-6-N-pyrrolidinyl-sulfonylphenol hergestellt und dieses gegebenenfalls in ein physiologisch verträgliches Säureadditionssalz überführt wird.

7. Verfahren zur Herstellung von Verbindungen der Formel II

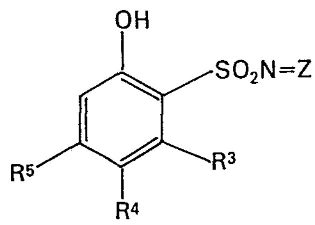

(II)

worin $R^3$ bis $R^5$ die in Anspruch 1 angegebenen Bedeutungen haben und Z für die gemäss Anspruch 1a definierte Sulfonamidschutzgruppe steht, dadurch gekennzeichnet, dass man Verbindungen der Formel XII

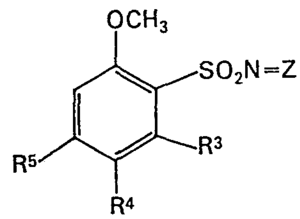

(XII)

in der $R^3$ bis $R^5$ und Z die obengenannte Bedeutung haben, einer Etherspaltung unterwirft.

**Claims for the Contracting states: BE, CH, DE, FR, GB, IT, LI, NL**

1. A 2-aminomethylphenol of the formula I

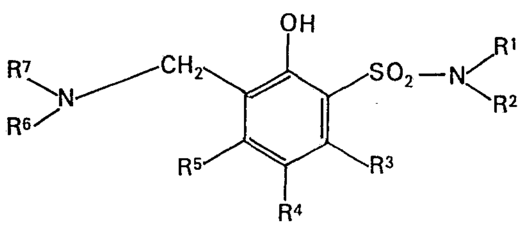

(I)

in which $R^1$ and $R^2$ are identical or different and represent hydrogen, alkyl having 1 to 8 carbon atoms, alkenyl having 2 to 8 carbon atoms, alkynyl having 2 to 8 carbon atoms, cycloalkyl having 3 to 12 carbon atoms and up to 8 ring members or benzyl which is optionally substituted by alkyl having 1 to 4 carbon atoms, alkoxy having 1 to 4 carbon atoms or halogen,
$R^3$ and $R^5$ are identical or different and denote hydrogen, halogen, alkyl having 1 or 2 carbon atoms or alkoxy having 1 or 2 carbon atoms,
$R^4$ denotes halogen, alkyl having 1 to 12 carbon atoms or cycloalkyl having 3 to 12 carbon atoms and up to 8 ring members, and
$R^6$ and $R^7$ are identical or different and represent hydrogen or alkyl having 1 to 4 carbon atoms,
it being possible for the radicals $R^1$ and $R^2$, $R^6$ and $R^7$ and/or two of the radicals $R^3$, $R^4$ and $R^5$ to form a $-[CH_2]_m$-chain in which m is 3 to 6 and which is optionally substituted by 1 or 2 methyl groups and which, in the case of the radicals $R^1$, $R^2$, $R^6$ and $R^7$, can also be interrupted by 1 or 2 oxygen atoms, sulfur atoms and/or imino groups, and to physiologically acceptable salts thereof.

2. A compound of the formula I as claimed in claim 1, wherein $R^1$ and $R^2$ are identical or different and represent hydrogen, alkyl having 1 to 4 carbon atoms, alkenyl or alkynyl having 2 to 4 carbon atoms and benzyl which is optionally substituted by methyl, methoxy or halogen, $R^3$ and $R^5$ have the meaning mentioned in claim 1, $R^4$ is halogen, alkyl having 1 to 8 carbon atoms, cycloalkyl having 3 to 10 carbon atoms and up to 7 ring members, and $R^6$ and $R^7$ are identical or different and denote hydrogen or alkyl having 1 or 2 carbon atoms, it being possible for the radicals $R^1$ and $R^2$, $R^6$ and $R^7$ and/or two of the radicals $R^3$, $R^4$ and $R^5$ to form a polymethylene chain defined above.

3. 2-Aminomethyl-4-(1,1-dimethylethyl)-6-sulfamoylphenol and physiologically acceptable acid addition salts thereof.

4. 2-Aminomethyl-4-isopropyl-6-sulfamoylphenol and physiologically acceptable acid addition salts thereof.

5. 2-Aminomethyl-4-(1-methylpropyl)-6-sulfamoylphenol and physiologically acceptable acid addition salts thereof.

6. 2-Aminomethyl-4-(1,1-dimethylethyl)-6-N-pyrrolidinylsulfonylphenol and physiologically acceptable acid addition salts thereof.

7. A process for the preparation of a 2-aminomethylphenol as claimed in claim 1, which comprises

a) reacting a phenol of the formula II

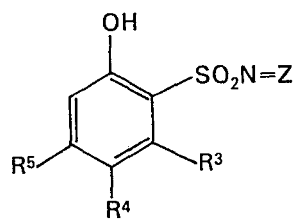

(II)

in which the radicals $R^3$ to $R^5$ have the meanings mentioned in claim 1 and Z either represents either the two radicals $R^1$ and $R^2$, which have the meanings mentioned in claim 1 with the exception of that of hydrogen, or represents a sulfonamide protective group of the formula III

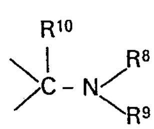

(III)

in which the radicals $R^{10}$ denote hydrogen or alkyl having 1 to 4 carbon atoms and $R^8$ and $R^9$ denote alkyl having 1 to 4 carbon atoms, with an N-hydroxymethylcarboxamide of the formula IV

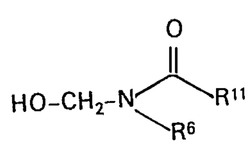

(IV)

in which $R^6$ has the meaning defined in claim 1 and the radical $CO-R^{11}$ represents an acyl group in which $R^{11}$ can be hydrogen, optionally halogen-

substituted alkyl having 1 to 4 carbon atoms or aryl having 6 to 10 carbon atoms, or in which $R^6$ and the radical CO-$R^{11}$ together form the o-phthaloyl radical, to give a compound of the formula V

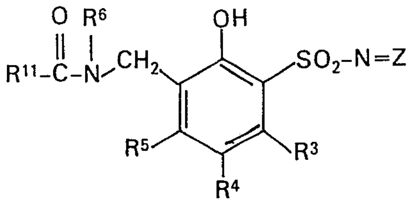

(V)

and splitting off by hydrolysis the acyl radical CO-$R^{11}$ and also, if appropriate, the sulfonamide protective group Z,

b) reacting a phenol of the formula II in which the radicals $R^3$, $R^4$ and $R^5$ and also Z have the meanings mentioned in claim 7a, with an amine of the formula VI

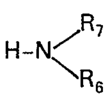

(VI)

in which $R^7$ and $R^6$ have the meaning mentioned in claim 1 with the exception of that of hydrogen, in the presence of formaldehyde, and, if appropriate, splitting off by hydrolysis the sulfonamide protective group Z,

c) chlorosulfonating a phenol of the formula VII

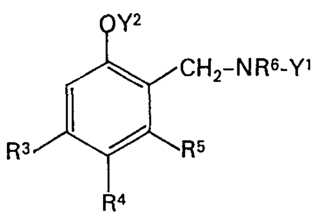

(VII)

in which $R^3$ to $R^6$ have the meanings mentioned in claim 1 and $Y^1$ represents the radical $R^{11}$ -CO- in which $R^{11}$ has the meaning mentioned above, and $Y^2$ denotes either hydrogen or $Y^1$, to give a compound of the formula IX

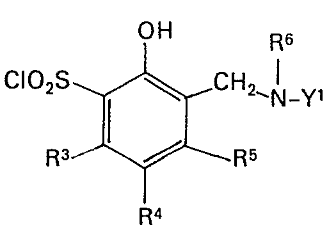

(IX)

and reacting the latter with an amine of the formula X

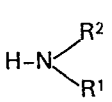

(X)

in which $R^1$ and $R^2$ have the meanings mentioned in claim 1, to give a compound of the formula XI

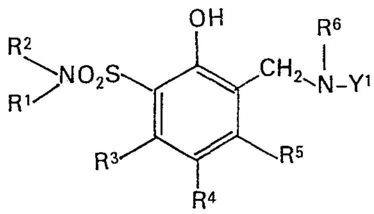

(XI)

and splitting off the radical $Y^1$ by hydrolysis from this compound, or

d) reacting a compound of the formula XVIII

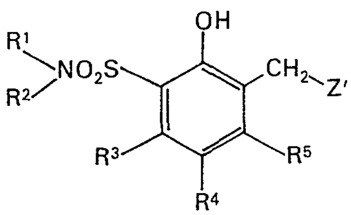

(XVIII)

in which $R^1$ to $R^5$ have the meanings mentioned in claim 1, it being, however, also possible for $R^1$ and $R^2$ together to represent the sulfonamide protective group Z mentioned in process variant a, and Z′ represents a leaving group, with an amine of the formula H-N = Y′ in which Y′ either represents the radicals $R^6$ and $R^7$ defined above or represents one of the radicals $R^6$ or $R^7$ and an amine protective group, or Y′ alone denotes an amine protective group, to give a compound of the formula XIX

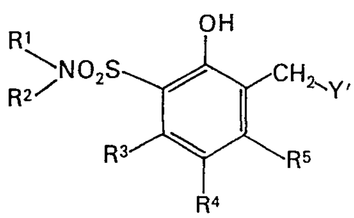

(XIX)

and splitting off from the latter, if appropriate, the amine protective group and/or the sulfonamide protective group, by hydrolysis or hydrogenolysis.

8. Pharmaceutical formulations based on a compound as claimed in claim 1.

9. A process for the preparation of a pharmaceutical formulation as claimed in claim 8, which comprises converting a compound as claimed in claim 1, if appropriate together with pharmacologically acceptable excipients and/or stabilizers, into a suitable form for administration.

10. Compounds as claimed in claim 1 for application in the treatment of edema diseases and of hypertension.

11. A compound of the formula II

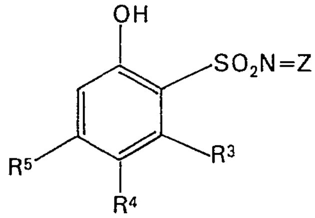

(II)

in which $R^3$ to $R^5$ have the meanings indicated in claim 1 and Z represents the sulfonamide protective group defined in accordance with claim 7a.

**Claims for the Contracting state: AT**

1. A process for the production of 2-aminomethylphenols of the formula I

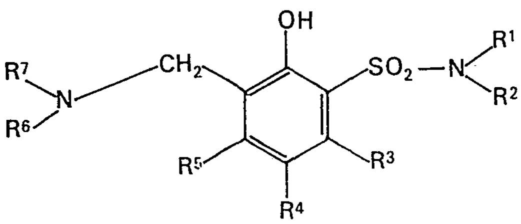

(I)

in which $R^1$ and $R^2$ are identical or different and represent hydrogen, alkyl having 1 to 8 carbon atoms, alkenyl having 2 to 8 carbon atoms, alkynyl having 2 to 8 carbon atoms, cycloalkyl having 3 to 12 carbon atoms and up to 8 ring members or benzyl which is optionally substituted by alkyl having 1 to 4 carbon atoms, alkoxy having 1 to 4 carbon atoms or halogen,
$R^3$ and $R^5$ are identical or different and denote hydrogen, halogen, alkyl having 1 or 2 carbon atoms or alkoxy having 1 or 2 carbon atoms,
$R^4$ denotes halogen, alkyl having 1 to 12 carbon atoms or cycloalkyl having 3 to 12 carbon atoms and up to 8 ring members, and
$R^6$ and $R^7$ are identical or different and represent hydrogen or alkyl having 1 to 4 carbon atoms,
it being possible for the radicals $R^1$ and $R^2$, $R^6$ and $R^7$ and/or two of the radiclas $R^3$, $R^4$ and $R^5$ to form a $-[CH_2]_m$-chain in which m is 3 to 6 and which is optionally substituted by 1 or 2 methyl groups and which, in the case of the radicals $R^1$, $R^2$, $R^6$ and $R^7$, can also be interrupted by 1 or 2 oxygen atoms, sulfur atoms and/or imino groups, and to physiologically acceptable salts thereof, which comprises

a) reacting a phenol of the formula II

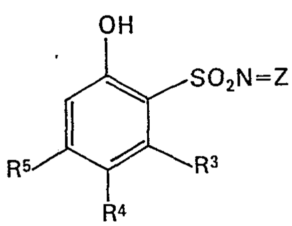

(II)

in which the radicals $R^3$ to $R^5$ have the meanings mentioned above and Z either represents either the two radicals $R^1$ and $R^2$, which have the meanings mentioned above with the exception of that of hydrogen, or represents a sulfonamide protective group of the formula III

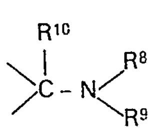

(III)

in which the radicals $R^{10}$ denote hydrogen or alkyl having 1 to 4 carbon atoms and $R^8$ and $R^9$ denote alkyl having 1 to 4 carbon atoms, with an N-hydroxymethylcarboxamide of the formula IV

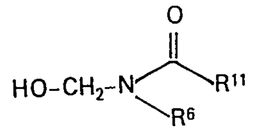

(IV)

in which $R^6$ has the meaning defined above and the radical CO-$R^{11}$ represents an acyl group in which $R^{11}$ can be hydrogen, optionally halogen-substituted alkyl having 1 to 4 carbon atoms or aryl having 6 to 10 carbon atoms, or in which $R^6$ and the radical CO-$R^{11}$ together form the o-phthaloyl radical, to give a compound of the formula V

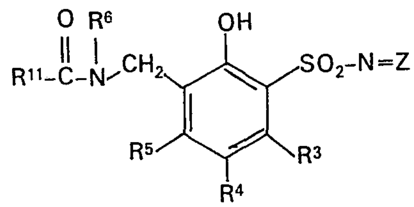

(V)

and splitting off by hydrolysis the acyl radical CO-$R^{11}$ and also, if appropriate, the sulfonamide protective group Z,

b) reacting a phenol of the formula II in which the radicals $R^3$, $R^4$ and $R^5$ and also Z have the meanings mentioned in variant a, with an amine of the formula VI

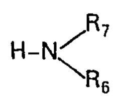

(VI)

in which $R^7$ and $R^6$ have the meaning mentioned above with the exception of that of hydrogen, in the presence of formaldehyde, and, if appropriate, splitting off by hydrolysis the sulfonamide protective group Z,

c) chlorosulfonating a phenol of the formula VII

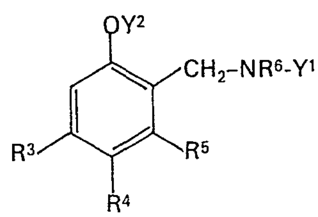

(VII)

in which $R^3$ to $R^6$ have the meanings above and $Y^1$ represents the radical $R^{11}$-CO- in which $R^{11}$ has the meaning mentioned above, and $Y^2$ denotes either hydrogen or $Y^1$, to give a compound of the formula IX

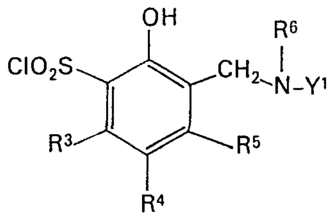

(IX)

and reacting the latter with an amine of the formula X

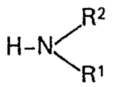

(X)

in which $R^1$ and $R^2$ have the meanings mentioned above, to give a compound of the formula XI

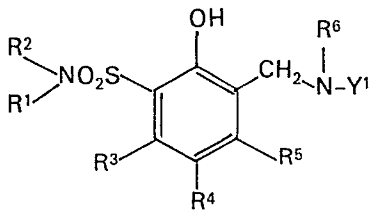

(XI)

and splitting off the radical $Y^1$ by hydrolysis from this compound, or

d) reacting a compound of the formula XVIII

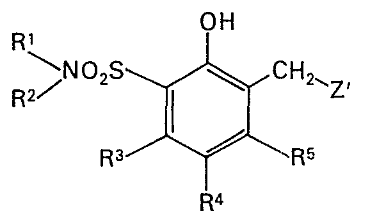

(XVIII)

in which $R^1$ to $R^5$ have the meanings mentioned above, it being, however, also possible for $R^1$ and $R^2$ together to represent the sulfonamide protective group Z mentioned in process variant a, and Z′ represents a leaving group, with an amine of the formula H-N = Y′ in which Y′ either represents the radicals $R^6$ and $R^7$ defined above or represents one of the radicals $R^6$ or $R^7$ and an amine protective group, or Y′ alone denotes an amine protective group, to give a compound of the formula XIX

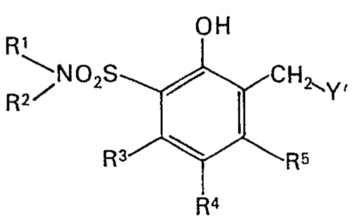

(XIX)

and splitting off from the latter, if appropriate, the amine protective group and/or the sulfonamide protective group, by hydrolysis or hydrogenolysis and optionally converting compounds I into their salts.

2. A process as claimed in claim 1, wherein $R^1$ and $R^2$ are identical or different and represents hydrogen, alkyl having 1 to 4 carbon atoms, alkenyl or alkynyl having 2 to 4 carbon atoms, and benzyl which is optionally substituted by methyl, methoxy or halogen,
$R^3$ and $R^5$ have the meaning mentioned in claim 1, $R^4$ is halogen, alkyl having 1 to 8 carbon atoms, cycloalkyl having 3 to 10 carbon atoms and up to 7 ring members, and $R^6$ and $R^7$ are identical or different and denote hydrogen or alkyl having 1 or 2 carbon atoms,
it being possible for the radicals $R^1$ and $R^2$, $R^6$ and $R^7$ and/or two of the radicals $R^3$, $R^4$ and $R^5$ to form a polymethylene chain defined above.

3. A process according to claim 1 characterized in that 2-aminomethyl-4-(1,1dimethylethyl)-6-sulfamoyl-phenol is produced and is optionally converted into a physiologically acceptable acid addition salt thereof.

4. A process according to claim 1, characterized in that 2-aminomethyl-4-isopropyl-6-sulfamoylphenol is produced and is optionally converted into a physiologically acceptable acid addition salt thereof.

5. A process according to claim 1 characterized in that 2-aminomethyl-4-(1-methylpropyl)-6-sulfamoylphenol is produced and is optionally converted into a physiologically acceptable acid addition salt thereof.

6. A process according to claim 1 characterized in that 2-aminomethyl-4-(1,1-dimethylethyl)-6-N-pyrrolidinylsulfonylphenol is produced and is optionally converted into a physiologically acceptable acid addition salt thereof.

7. A process for the preparation of a compound of the formula II

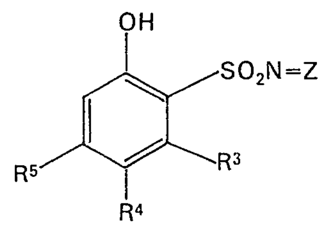

(II)

in which $R^3$ to $R^5$ have the meanings indicated in claim 1 and Z represents the sulfonamide protective group defined in accordance with claim 1a, characterized in that a compound of the formula XII

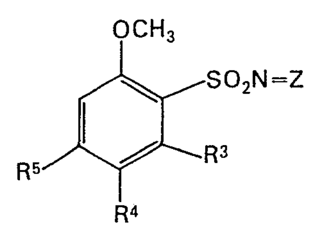

(XII)

wherein $R^3$ to $R^5$ and Z have the above-mentioned meanings are subjected to an ether cleavage.

**Revendications pour les états contractants: BE, CH, DE, FR, GB, IT, LI, NL**

1. 2-Aminométhylphénols de formule I

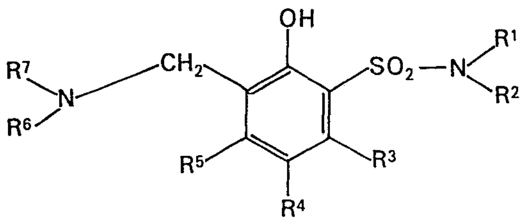

(I)

dans laquelle:
$R^1$ et $R^2$ sont identiques ou différents et représentent l'hydrogène, un groupe alkyle ayant de 1 à 8 atomes de carbone, alcényle ayant de 2 à 8 atomes de carbone, alcynyle ayant de 2 à 8 atomes de carbone, cycloalkyle ayant de 3 à 12 atomes de carbone et jusqu'à 8 chaînons dans le noyau ou un groupe benzyle, qui est éventuellement substitué par un groupe alkyle ayant de 1 à 4 atomes de carbone, un groupe alcoxy ayant de 1 à 4 atomes de carbone ou par un halogène,
$R^3$ et $R^5$ sont identiques ou différents et représentent l'hydrogène, un halogène, un groupe alkyle ayant 1 ou 2 atomes de carbone ou alcoxy ayant 1 ou 2 atomes de carbone,
$R^4$ représente un halogène, un groupe alkyle ayant de 1 à 12 atomes de carbone ou cycloalkyle ayant de 3 à 12 atomes de carbone et jusqu'à 8 chaînons dans le noyau, et
$R^6$ et $R^7$ sont identiques ou différents et représentent l'hydrogène, ou un groupe alkyle ayant de 1 à 4 atomes de carbone,
les radicaux $R^1$ et $R^2$, $R^6$ et $R^7$ et/ou deux des radicaux $R^3$, $R^4$ et $R^5$ pouvant former une chaîne $-[CH_2]-_m$ avec $m = 3$ à 6, qui est éventuellement substituée par 1 ou 2 groupes méthyle et qui dans le cas des radicaux $R^1$, $R^2$, $R^6$ et $R^7$ peut également être interrompue par 1 ou 2 atomes d'oxygène, de soufre et/ou par des groupes imino, ainsi que leurs sels physiologiquement acceptables.

2. Composés de formule I selon la revendication 1, caractérisés en ce qui:
$R^1$ et $R^2$ sont identiques ou différents et représentent l'hydrogène, un groupe alkyle ayant de 1 à 4 atomes de carbone, alcényle ou alcynyle ayant de 2 à 4 atomes de carbone, ou benzyle qui peut éventuellement être substitué par un groupe méthyle, méthoxy ou par un halogène,
$R^3$ et $R^5$ ont les significations indiquées dans la revendication 1,
$R^4$ représente un halogène, un groupe alkyle ayant de 1 à 8 atomes de carbone, cycloalkyle ayant de 3 à 10 atomes de carbone et jusqu'à 7 chaînons dans le noyau, et
$R^6$ et $R^7$ sont identiques ou différents et représentent l'hydrogène ou un groupe alkyle ayant 1 ou 2 atomes de carbone,
les radicaux $R^1$ et $R^2$, $R^6$ et $R^7$ et/ou deux des radicaux $R^3$, $R^4$ et $R^5$ peuvent former une chaîne polyméthylénique définie dans la revendication 1.

3. 2-Aminométhyl-4-(1,1-diméthyléthyl)-6-sulfamoylphénol et ses sels d'addition avec des acides physiologiquement acceptables.

4. 2-Aminométhyl-4-isopropyl-6-sulfamoylphénol et ses sels d'addition avec des acides physiologiquement acceptables.

5. 2-Aminométhyl-4-(1-méthylpropyl)-6-sulfamoylphénol et ses sels physiologiquement acceptables.

6. 2-Aminométhyl-4-(1,1-diméthyléthyl)-6-N-pyrrolidinylsulfonylphénol et ses sels d'addition avec des acides physiologiquement acceptables.

7. Procédé pour la préparation de 2-aminométhylphénols selon la revendication 1, caractérisé en ce que:
a) on fait réagir un phénol de formule II

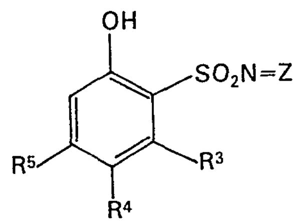

(II)

dans laquelle les radicaux $R^3$ à $R^5$ ont les significations indiquées dans la revendication 1, et Z représente, soit les deux radicaux $R^1$ et $R^2$ qui ont les significations indiquées dans la revendication 1 à l'exception de l'hydrogène, soit un groupe sulfonamido-protecteur de formule III

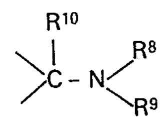

(III)

dans laquelle les radicaux $R^{10}$ représentent l'hydrogène ou un groupe alkyle ayant de 1 à 4 atomes de carbone, et $R^8$ et $R^9$ un groupe alkyle ayant de 1 à 4 atomes de carbone,
avec un N-hydroxyméthylcarboxamide de formule IV

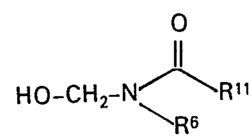

(IV)

dans laquelle $R^6$ a la signification indiquée dans la revendication 1 et le radical $CO-R^{11}$ représente un groupe acyle dans lequel $R^{11}$ peut etre l'hydrogène, un groupe alkyle éventuellement substitué par un halogène, ayant de 1 à 4 atomes de carbone, ou un groupe aryle ayant de 6 à 10 atomes de carbone, ou dans laquelle $R^6$ et le radical $CO-R^{11}$ forment ensemble le radical o-phtaloyle, pour obtenir des composés de formule V

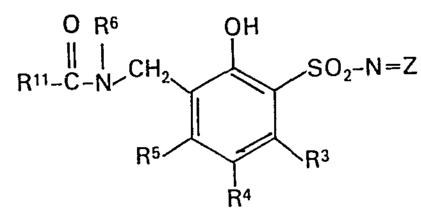

(V)

et ont élimine le radical acyle CO-$R^{11}$ ainsi qu'éventuellement le groupe sulfonamido-protecteur Z par hydrolyse,

b) on fait réagir un phénol de formule II, dans lequel les radicaux $R^3$, $R^4$ et $R^5$ ainsi que Z ont les significations indiquées dans la revendication 7a, avec une amine de formule VI

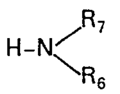

(VI)

dans laquelle $R^7$ et $R^6$ ont les significations indiquées dans la revendication 1, à l'exception de l'hydrogène, en présence de formaldéhyde et éventuellement ont élimine par hydrolyse le groupe sulfonamido-protecteur Z,

c) on chlorosulfone des phénols de formule VII

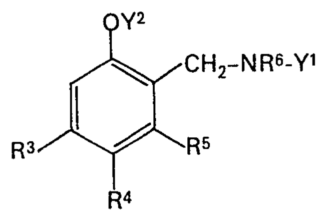

(VII)

dans laquelle $R^3$ à $R^6$ ont les significations indiquées dans la revendication 1 et $Y^1$ représente le radical $R^{11}$-CO-, $R^{11}$ ayant la signification indiquée ci-dessus, et $Y^2$ représente l'hydrogène ou $Y^1$, pour obtenir des composés de formule IX

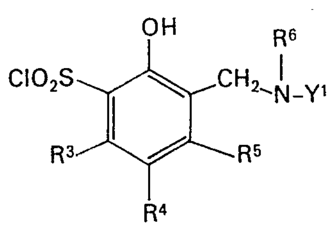

(IX)

et ont fait réagir ceux-ci avec des amines de formule X

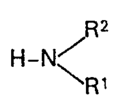

(X)

dans laquelle $R^1$ et $R^2$ ont les significations indiquées dans la revendication 7, pour obtenir des composés de formule XI

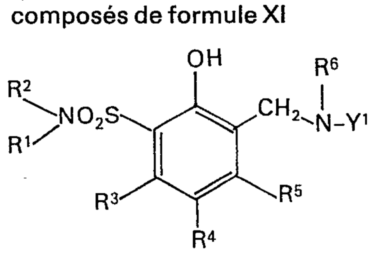

(XI)

puis, on élimine le radical $Y^1$ de ces composés par hydrolyse, ou

d) on fait réagir des composés de formule XVIII

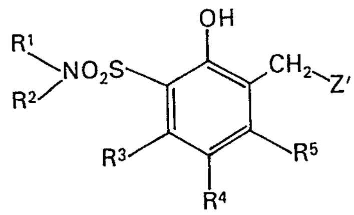

(XVIII)

dans laquelle $R^1$ à $R^5$ ont les significations indiquées dans la revendication 1, $R^1$ et $R^2$ pouvant également former ensemble le groupe sulfonamido-protecteur Z mentionné pour la variante opératoire a et Z' représente un groupe labile, avec des amines de formule H-N = Y', dans lesquelles Y' représente les radicaux $R^6$ et $R^7$ définis ci-dessus ou l'un des radicaux $R^6$ ou $R^7$ et un groupe amino-protecteur ou Y' représente seul un groupe amino-protecteur, pour obtenir des composés de formule XIX

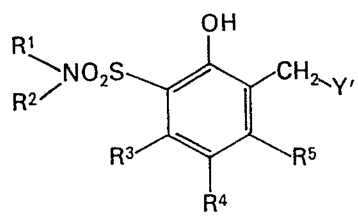

(XIX)

et ont élimine de ces composés éventuellement le groupe amino-protecteur et/ou le groupe sulfonamido-protecteur, par hydrolyse ou hydrogénolyse.

8. Compositions pharmaceutiques contenant un composé selon la revendication 1.

9. Procédé pour la préparation d'une composition pharmaceutique selon la revendication 8, caractérisé en ce qu'on met un composé selon la revendication 1 sous une forme d'administration appropriée, éventuellement avec des véhicules pharmacologiquement acceptables et/ou des stabilisants.

10. Composés selon la revendication 1, utilisables pour le traitement des maladies à œdèmes et de l'hypertonie.

11. Composés de formule II

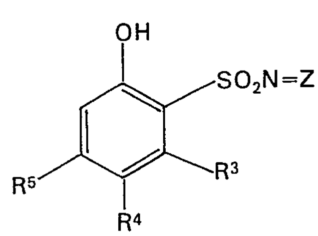

(II)

dans laquelle $R^3$ à $R^5$ ont les significations indiquées dans la revendication 1 et Z représente le groupe sulfonamido-protecteur défini selon la revendication 7a.

**Revendications pour l'état contractant: AT**

1. Procédé pour la préparation de 2-aminométhylphénols de formule I

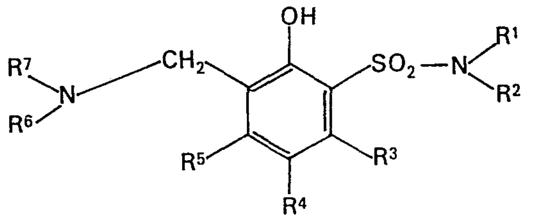

(I)

dans laquelle,
$R^1$ et $R^2$ sont identiques ou différents et représentent l'hydrogène, un groupe alkyle ayant de 1 à 8 atomes de carbone, alcényle ayant de 2 à 8 atomes de carbone, alcynyle ayant de 2 à 8 atomes de carbone, cycloalkyle ayant de 3 à 12 atomes de carbone et jusqu'à 8 chaînons dans le noyau, ou un groupe benzyle, qui est éventuellement substitué par un groupe alkyle ayant de 1 à 4 atomes de carbone, alcoxy ayant de 1 à 4 atomes de carbone ou par un halogène,
$R^3$ et $R^5$ sont identiques ou différents et représentent l'hydrogène, un halogène, un groupe alkyle ayant 1 ou 2 atomes de carbone ou alcoxy ayant de 1 ou 2 atomes de carbone,
$R^4$ représente un halogène, un groupe alkyle ayant de 1 à 12 atomes de carbone ou cycloalkyle ayant de 3 à 12 atomes de carbone et jusqu'à 8 chaînons dans le noyau, et
$R^6$ et $R^7$ sont identiques ou différents et représentent l'hydrogène ou un groupe alkyle ayant de 1 à 4 atomes de carbone,
les radicaux $R^1$ et $R^2$, $R^6$ et $R^7$ et/ou deux des radicaux $R^3$, $R^4$ et $R^5$ pouvant former une chaîne $-[CH_2]_m$ avec $m = 3$ à 6, qui peut être substituée par 1 ou 2 groupes méthyle et qui, dans les cas des radicaux $R^1$, $R^2$, $R^6$ et $R^7$, peut également être interrompue par 1 ou 2 atomes d'oxygène, de soufre et/ou des groupes imino,
ainsi que leurs sels physiologiquement acceptables, lequel procédé est caractérisé en ce que:

a) on fait réagir un phénol de formule II

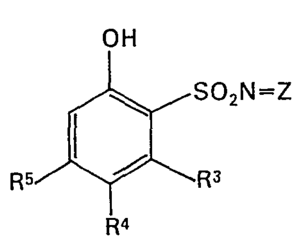

(II)

dans laquelle les radicaux $R^3$ à $R^5$ ont les significations indiquées ci-dessus et Z représentent soit les deux radicaux $R^1$ et $R^2$ qui ont les significations indiquées ci-dessus à l'exception de l'hydrogène, ou un groupe sulfonamido-protecteur de formule III

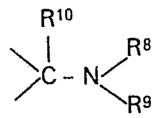

(III)

dans laquelle les radicaux $R^{10}$ représentent l'hydrogène ou un groupe alkyle ayant de 1 à 4 atomes de carbone et $R^8$ et $R^9$ un groupe alkyle ayant de 1 à 4 atomes de carbone,
avec un N-hydroxyméthylcarboxamide de formule IV

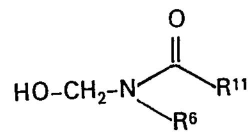

(IV)

dans laquelle $R^6$ a la signification indiquée ci-dessus et le radical $CO\text{-}R^{11}$ représente un groupe acyle dans lequel $R^{11}$ peut être l'hydrogène, un groupe alkyle éventuellement substitué par un halogène ayant de 1 à 4 atomes de carbone ou aryle ayant de 6 à 10 atomes de carbone, ou dans laquelle $R^6$ et le radical $CO\text{-}R^{11}$ forment ensemble le groupe o-phtaloyle, pour obtenir des composés de formule V

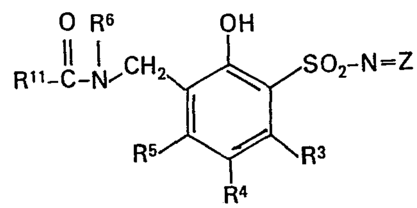

(V)

et on élimine le radical acyle $CO\text{-}R^{11}$ ainsi qu'éventuellement le groupe sulfonamido-protecteur Z par hydrolyse,

b) on fait réagir un phénol de formule II, dans lequel les radicaux $R^3$, $R^4$ et $R^5$ ainsi que Z ont les significations indiquées ci-dessus,
avec une amine de formule IV

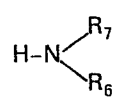

(VI)

dans laquelle $R^7$ et $R^6$ ont les significations indiquées ci-dessus, à l'exception de l'hydrogène, en présence de formaldehyde, et éventuellement on élimine par hydrolyse le groupe sulfonamido-protecteur Z.

c) on chlorosulfone des phénols de formule VII

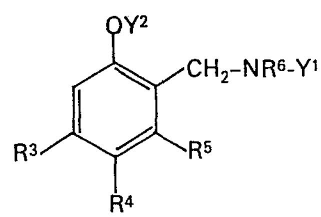

(VII)

dans laquelle $R^3$ à $R^6$ ont les significations indiquées ci-dessus et $Y^1$ représente le radical $R^{11}$-CO, $R^{11}$ ayant la signification indiquée ci-dessus et $Y^2$ représente, soit l'hydrogène, soit $Y^1$, pour obtenir des composés de formule IX

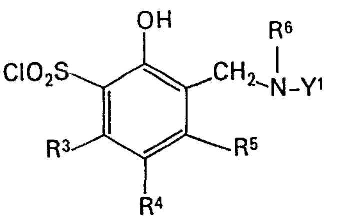

(IX)

et on fait réagir ces derniers avec des amines de formule X

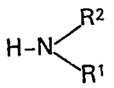

(X)

dans laquelle $R^1$ et $R^2$ ont les significations mentionées ci-dessus, pour obtenir des composés de formule XI

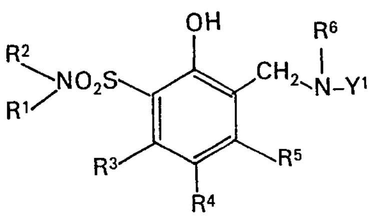

(XI)

et on élimine de ces composés le radical $Y^1$ par hydrolyse, ou

d) on fait réagir des composés de formule XVIII

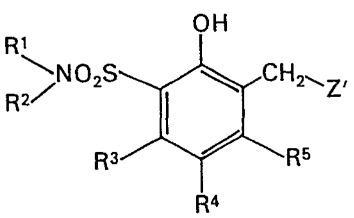

(XVIII)

dans laquelle $R^1$ à $R^5$ ont les significations indiquées ci-dessus, $R^1$ et $R^2$ pouvant également former ensemble le groupe sulfonamido-protecteur Z mentionné pour la variante opératoire a, et Z' représente un groupe labile, avec des amines de formule H-N = Y', dans laquelle Y' est, soit les radicaux $R^6$ et $R^7$ définis ci-dessus, soit l'un des radicaux $R^6$ ou $R^7$ et un groupe amino-protecteur, ou Y' représente seul un groupe amino-protecteur, pour obtenir des composés de formule XIX

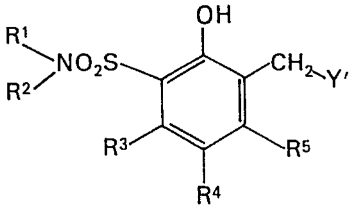

(XIX)

et on élimine de ces composés éventuellement le groupe amino-protecteur et/ou le groupe sulfonamido-portecteur par hydrolyse ou hydrogénolyse et on convertit éventuellement les composés de formule I obtenus en leurs sels.

2. Procédé selon la revendication 1, caractérisé en ce que l'on prépare des composés de formule I, dans lesquels,

$R^1$ et $R^2$ sont identiques ou différents et réprésentent l'hydrogène, un groupe alkyle ayant de 1 à 4 atomes de carbone, alcényle ou alcynyle ayant de 2 à 4 atomes de carbone et benzyle, qui est éventuellement substitué par un groupe méthyle, méthoxy, ou par un halogène,

$R^3$ et $R^5$ ont les significations indiquées dans la revendication 1,

$R^4$ représente un halogène, un groupe alkyle ayant de 1 à 8 atomes de carbone, cycloalkyle ayant de 3 à 10 atomes de carbone et jusqu'à 7 chaînons dans le noyau, et

$R^6$ et $R^7$ sont identiques ou différents et représentent l'hydrogène ou un groupe alkyle ayant 1 ou 2 atomes de carbone,

les radicaux $R^1$ et $R^2$, $R^6$ et $R^7$ et/ou deux des radicaux $R^3$, $R^4$ et $R^5$ pouvant former une chaîne polyméthylénique définie dans la revendication 1.

3. Procédé selon la revendication 1, caractérisé en ce qu'on prépare le 2-aminométhyl-4-(1,1-diméthyléthyl)-6-sulfamoylphénol et on convertit éventuellement ce dernier en un sel d'addition avec un acide physiologiquement acceptable.

4. Procédé selon la revendication 1, caractérisé en ce qu'on prépare le 2-aminométhyl-4-isopropyl-6-sulfamoylphénol et on convertit éventuellement ce dernier en un sel d'addition avec un acide physiologiquement acceptable.

5. Procédé selon la revendication 1, caractérisé en ce qu'on prépare le 2-aminométhyl-4-(1-méthylpropyl)-6-sulfamoylphénol et on convertit éventuellement ce dernier en un sel physiologiquement acceptable.

6. Procédé selon la revendication 1, caractérisé en ce qu'on prépare le 2-aminométhyl-4-(1,1-diméthyléthyl)-6-N-pyrrolidinyl-sulfonylphénol et on convertit éventuellement ce dernier en un sel d'addition avec un acide physiologiquement acceptable.

7. Procédé pour la préparation de composés de formule II

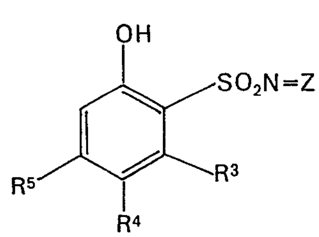

(II)

dans laquelle $R^3$ à $R^5$ ont les significations indiquées dans la revendication 1 et Z est le groupe sulfonamidoprotecteur défini selon la revendication 1a, caractérisé en ce qu'on soumet à une élimination du groupe éther des composés de formule XII

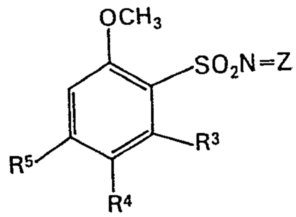

(XII)

dans laquelle $R^3$ à $R^5$ et Z ont les significations indiquées ci-dessus.